(19) Europäisches Patentamt
European Patent Office
Office européen des brevets

(11) EP 1 548 114 B1

(12) EUROPEAN PATENT SPECIFICATION

(45) Date of publication and mention of the grant of the patent:
**26.09.2012 Bulletin 2012/39**

(21) Application number: **03788072.1**

(22) Date of filing: **11.08.2003**

(51) Int Cl.:
***C12N 15/13*** *(2006.01)* ***C12P 21/02*** *(2006.01)*
***C07K 16/00*** *(2006.01)* ***A01K 67/027*** *(2006.01)*

(86) International application number:
**PCT/JP2003/010198**

(87) International publication number:
**WO 2004/016081 (26.02.2004 Gazette 2004/09)**

# (54) METHOD OF EXPRESSING GENE IN TRANSGENIC BIRDS USING RETROVIRUS VECTOR AND TRANSGENIC BIRDS THUS OBTAINED

VERFAHREN ZUR GENEXPRESSION IN TRANSGENEN VÖGELN UNTER VERWENDUNG EINES RETROVIRUSVEKTORS UND SO ERHALTENE TRANSGENE VÖGEL

TECHNIQUE D'EXPRESSION DE GENE DANS DES OISEAUX TRANSGENIQUES AU MOYEN DE VECTEUR RETROVIRAL ET OISEAUX TRANSGENIQUES AINSI OBTENUS

(84) Designated Contracting States:
**AT BE BG CH CY CZ DE DK EE ES FI FR GB GR HU IE IT LI LU MC NL PT RO SE SI SK TR**

(30) Priority: **13.08.2002 JP 2002236089**

(43) Date of publication of application:
**29.06.2005 Bulletin 2005/26**

(73) Proprietors:
- **KANEKA CORPORATION**
  **Osaka-shi, Osaka 530-8288 (JP)**
- **Nagoya Industrial Science Research Institute**
  **Nagoya-shi,**
  **Aichi 460-0008 (JP)**

(72) Inventors:
- **IIJIMA, Shinji**
  **Nagoya-shi, Aichi 468-0022 (JP)**
- **KAMIHIRA, Masamichi,**
  **603, Takaramanshonshirokicho**
  **Fukuoka-shi,**
  **Fukuoka 819-0395 (JP)**
- **NISHIJIMA, Kenichi,**
  **302, Kuzuokamanshon**
  **Nagoya-shi,**
  **Aichi 464-0804 (JP)**
- **ONO, Kenichiro**
  **Ina-shi,**
  **Nagano 396-0011 (JP)**

(74) Representative: **HOFFMANN EITLE**
**Patent- und Rechtsanwälte**
**Arabellastrasse 4**
**81925 München (DE)**

(56) References cited:
**EP-A1- 0 424 044** **WO-A1-02/47475**
**WO-A1-97/03555** **JP-A- 2002 176 880**

- **HARVEY A J ET AL: "Expression of exogenous protein in the egg white of transgenic chickens" NATURE BIOTECHNOLOGY, NATURE PUBLISHING GROUP, NEW YORK, NY, US, vol. 19, April 2002 (2002-04), pages 396-399, XP002973268 ISSN: 1087-0156**
- **THORAVAL: "GERMLINE TRANSMISSION OF EXOGENOUS GENE IN CHICKENS USING HELPER-FREE ECOTROPIC AVIAN LEUKOSIS VIRUS-BASED VECTORS" TRANSGENIC RESEARCH, LONDON, GB, vol. 4, no. 6, 1995, pages 369-376, XP008073707 ISSN: 0962-8819**
- **MIZUARAI S ET AL: "Production of transgenic quails with high frequency of germ-line transmission using VSV-G pseudotyped retroviral vector" BIOCHEMICAL AND BIOPHYSICAL RESEARCH COMMUNICATIONS, ACADEMIC PRESS, SAN DIEGO, CA, US, vol. 286, no. 3, August 2001 (2001-08), pages 456-463, XP002973599 ISSN: 0006-291X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- MOHAMMED S M ET AL: "Deposition of genetically engineered human antibodies into the egg yolk of hens" IMMUNOTECHNOLOGY, ELSEVIER SCIENCE PUBLISHERS BV, NL, vol. 4, no. 2, October 1998 (1998-10), pages 115-125, XP004153636 ISSN: 1380-2933
- ETCHES R J ET AL: "STRATEGIES FOR THE PRODUCTION OF TRANSGENIC CHICKENS" METHODS IN MOLECULAR BIOLOGY, HUMANA PRESS INC., CLIFTON, NJ, US, vol. 62, 1997, pages 433-450, XP000974906 ISSN: 1064-3745
- KAMIHIRA MASAMICHI ET AL: "High-level expression of single-chain Fv-Fc fusion protein in serum and egg white of genetically manipulated chickens by using a retroviral vector" JOURNAL OF VIROLOGY, vol. 79, no. 17, September 2005 (2005-09), pages 10864-10874, XP002474041 ISSN: 0022-538X
- MIZUARAI S. ET AL.: 'Production of transgenic quails with high frequency of germ-line transmission using VSV-G pseudotyped retroviral vector' BIOCHEM. BIOPHYS. RES. COMMUN. vol. 286, no. 3, August 2001, pages 456 - 463, XP002973599
- HARVEY A.J. ET AL.: 'Consistent production of transgenic chickens using replication-deficient retroviral vectors and high-throughput screening procedures' POULT. SCI. vol. 81, no. 2, February 2002, pages 202 - 212, XP002973600
- KEN'ICHIRO ONO ET AL.: 'Transgenic uzura ni okeru sonyu idenshi no genome kaiseki' THE SOCIETY OF CHEMICAL ENGINEERS, JAPAN SHUKI TAIKAI KENKYU HAPPYO KOEN YOSHISHU vol. 34, 31 August 2001, page 415, XP002975438
- POWERS D.B. ET AL.: 'Expression of single-chain Fv-Fc fusions in pichiapastris' J. IMMUNOL. METHODS vol. 251, May 2001, pages 123 - 135, XP004233079

SEQUENCE LISTING

<110> Kaneka Corporation,
Nagoya Industrial Science Research Institute (Chubu Technology Licensing Office)

<120> Method of expressing gene in transgenic birds using retrovirus vector and transgenic birds thus obtained

<130> T753/TRANS-1

<150> JP P2002-236089
<151> 2002-08-13

<160> 37

<210> 1
<211> 25
<212> DNA
<213> Artificial Sequence

<220>
<223> Designed sequence of a 5'-primer used for PCR amplification of the Miw promoter 5' region fragment

<400> 1
cggtctagag gaattcagtg gttcg    25

<210> 2
<211> 26
<212> DNA
<213> Artificial Sequence

<220>
<223> Designed sequence of a 3'-primer incorporating the BamH I recognition site at the 5' terminal used for PCR amplification of the Miw promoter 5' region fragment

<400> 2
ccaggatccg acgttgtaaa acgacg 26

<210> 3
<211> 28
<212> DNA
<213> Artificial Sequence

<220>
<223> Designed sequence of a 5'-primer incorporating the Hind III recognition site at the 5' terminal used for PCR amplification of the Miw promoter 3' region fragment

<400> 3
ccaaagcttg ccgcagccat tgcctttt 28

<210> 4
<211> 27
<212> DNA
<213> Artificial Sequence

<220>
<223> Designed sequence of a 3'-primer incorporating the Bln I recognition site at the 5' terminal used for PCR amplification of the Miw promoter 3' region fragment

<400> 4
atacctaggg gctggctgcg gaggaac 27

<210> 5
<211> 29
<212> DNA
<213> Artificial Sequence

<220>
<223> Designed sequence of a 5'-primer incorporating the Nhe I recognition site at the 5' terminal used for PCR amplification of the chicken β-actin promoter fragment lacking the intron

<400> 5
tttagctagc tgcagctcag tgcatgcac 29

<210> 6
<211> 27
<212> DNA
<213> Artificial Sequence

<220>
<223> Designed sequence of a 3'-primer incorporating the Xba I recognition site at the 5' terminal used for PCR amplification of the chicken β-actin promoter fragment lacking the intron

<400> 6
ataatctaga aacgcagcga ctcccgc 27

<210> 7
<211> 25
<212> DNA
<213> Artificial Sequence

<220>
<223> Designed sequence of a 5'-primer incorporating the Xho I recognition site at the 5' terminal used for PCR amplification of the coding fragment of the human antibody light chain κ constant region

<400> 7
atcctcgaga ggccaaagta cagtg 25

<210> 8
<211> 33
<212> DNA
<213> Artificial Sequence

<220>
<223> Designed sequence of a 3'-primer incorporating the BamH I recognition site at the 5' terminal used for PCR amplification of the coding fragment of the human antibody light chain κ constant region

<400> 8
cccggatccc taacactctc ccctgttgaa gct 33

<210> 9
<211> 48
<212> DNA
<213> Artificial Sequence

<220>
<223> Designed sequence of a 5'-primer incorporating the Not I recognition site at the 5' terminal used for PCR amplification of the coding fragment of the human antibody light chain variable region

<400> 9
agcggccgct acaggtgtcc actccgacat cgtgatgacc cagtctcc 48

<210> 10
<211> 34
<212> DNA
<213> Artificial Sequence

<220>
<223> Designed sequence of a 3'-primer incorporating the Xho I recognition site at the 5' terminal used for PCR amplification of the coding fragment of the human antibody light chain variable region

<400> 10
cctctcgagg atagaagtta ttcagcaggc acac 34

<210> 11
<211> 32
<212> DNA
<213> Artificial Sequence

<220>
<223> Designed sequence of a 5'-primer incorporating the Xho I recognition site at the 5' terminal used for PCR amplification of the coding fragment of the human antibody heavy chain μ constant region

<400> 11
acctcgagcg tggccgttgg ctgcctcgca ca 32

<210> 12
<211> 32
<212> DNA
<213> Artificial Sequence

<220>
<223> Designed sequence of a 3'-primer incorporating the Hind III recognition site at the 5' terminal used for PCR amplification of the coding fragment of the human antibody heavy chain μ constant region

<400> 12
actaagctta cgttgtacag ggtgggttta cc 32

<210> 13
<211> 48
<212> DNA
<213> Artificial Sequence

<220>
<223> Designed sequence of a 5'-primer incorporating the Not I recognition site at the 5' terminal used for PCR amplification of the coding fragment of the human antibody heavy chain variable region

<400> 13
agcggccgct acaggtgtcc actccgaggt gcagctggtg gagtctgg 48

<210> 14
<211> 36
<212> DNA
<213> Artificial Sequence

<220>
<223> Designed sequence of a 3'-primer incorporating the Xho I recognition site at the 5' terminal used for PCR amplification of the coding fragment of the human antibody heavy chain variable region

<400> 14
cacgctcgag gtatccgacg gggaattctc acagga 36

<210> 15
<211> 49
<212> DNA
<213> Artificial Sequence

<220>
<223> Designed sequence of a 5'-primer incorporating the Hind III recognition site at the 5' terminal used for DNA polymerase reaction to construct the coding fragment of the human epidermal growth factor receptor transmembrane region

<400> 15
cccaagcttg atctccactg ggatggtggg ggccctcctc ttgctgctg 49

<210> 16
<211> 78
<212> DNA
<213> Artificial Sequence

<220>
<223> Designed sequence of a 3'-primer incorporating the BamH I recognition site at the 5' terminal used for DNA polymerase reaction to construct the coding fragment of the human epidermal growth factor receptor transmembrane region

<400> 16

cccggatcct cagtcaaggc gccttcgcat gaagaggccg atccccaggg ccaccaccag 60
cagcaagagg agggcccc 78

<210> 17
<211> 31
<212> DNA
<213> Artificial Sequence

<220>
<223> Designed oligonucleotide used for site-directed mutagenesis to generate the Nar I recognition site at the 3' terminal of the coding fragment of the human antibody light chain variable region

<400> 17
tgaagacaga tggcgccgcc acagttcgtt t 31

<210> 18
<211> 30
<212> DNA
<213> Artificial Sequence

<220>
<223> Designed oligonucleotide used for site-directed mutagenesis to generate the BamH I recognition site at the 3' terminal of the coding fragment of the human antibody heavy chain variable region

<400> 18
tggggcggat gcggatcctg aggagacggt 30

<210> 19
<211> 30
<212> DNA
<213> Artificial Sequence

<220>
<223> Designed sequence of a 5'-primer incorporating the Not I recognition site at the 5' terminal used for PCR amplification of the coding fragment of the mouse antibody light chain variable region

<400> 19
cgcggccgcc tcagggaaag tttgaagatg 30

<210> 20
<211> 36
<212> DNA
<213> Artificial Sequence

<220>
<223> Designed sequence of a 3'-primer incorporating the Nar I recognition site at the 5' terminal used for PCR amplification of the coding fragment of the mouse antibody light chain variable region

<400> 20
cggcgccgcc acagtccgtt ttatttccag cttggt 36

<210> 21
<211> 30
<212> DNA

<213> Artificial Sequence

<220>
<223> Designed sequence of a 5'-primer incorporating the Not I recognition site at the 5' terminal used for PCR amplification of the coding fragment of the mouse antibody heavy chain variable region

<400> 21
cgcggccgcg aacacggamc cctcaccatg 30

<210> 22
<211> 28
<212> DNA
<213> Artificial Sequence

<220>
<223> Designed sequence of a 3'-primer incorporating the BamH I recognition site at the 5' terminal used for PCR amplification of the coding fragment of the mouse antibody heavy chain variable region

<400> 22
cggatcctgc agagacagtg accagagt 28

<210> 23
<211> 18
<212> DNA
<213> Artificial Sequence

<220>
<223> Designed sequence of a 5'-primer used for PCR amplification of the coding fragment of the human antibody heavy chain γ1 constant region

<400> 23
caagcttcaa gggcccat 18

<210> 24
<211> 19
<212> DNA
<213> Artificial Sequence

<220>
<223> Designed sequence of a 3'-primer used for PCR amplification of the coding fragment of the human antibody heavy chain γ1 constant region

<400> 24
atttacccgg agacaggga 19

<210> 25
<211> 30
<212> DNA
<213> Artificial Sequence

<220>
<223> Designed sequence of a 5'-primer incorporating the BamH I recognition site at the 5' terminal used for PCR amplification of the coding fragment of the human antibody heavy chain γ1 constant region

<400> 25
ataggatccg ctagcttcaa gggcccatcg 30

<210> 26

<211> 30
<212> DNA
<213> Artificial Sequence

<220>
<223> Designed sequence of a 3'-primer incorporating the Hind III recognition site at the 5' terminal used for PCR amplification of the coding fragment of the human antibody heavy chain γ1 constant or Fc region

<400> 26
agcaagcttt catttacccg gagacaggga 30

<210> 27
<211> 28
<212> DNA
<213> Artificial Sequence

<220>
<223> Designed sequence of a 5'-primer incorporating the Sal I recognition site at the 5' terminal used for PCR amplification of the chicken β-actin promoter fragment lacking the intron

<400> 27
acgcgtcgac gtgcatgcac gctcattg 28

<210> 28
<211> 26
<212> DNA
<213> Artificial Sequence

<220>
<223> Designed sequence of a 3'-primer incorporating the Sal I recognition site at the 5' terminal used for PCR amplification of the chicken β-actin promoter fragment lacking the intron

<400> 28
acgcgtcgac aacgcagcga ctcccg 26

<210> 29
<211> 30
<212> DNA
<213> Artificial Sequence

<220>
<223> Designed sequence of a 5'-primer incorporating the Sal I recognition site at the 5' terminal used for PCR amplification of the coding fragment of the antibody κ light chain

<400> 29
aatgtcgaca tggtgtccac ttctcagctc 30

<210> 30
<211> 30
<212> DNA
<213> Artificial Sequence

<220>
<223> Designed sequence of a 3'-primer incorporating the Sal I recognition site at the 5' terminal used for PCR amplification of the coding fragment of the antibody κ light chain

<400> 30
ttcgtcgacc taacactctc ccctgttgaa 30

<210> 31
<211> 28
<212> DNA
<213> Artificial Sequence

<220>
<223> Designed sequence of a 5'-primer incorporating the Sal I recognition site at the 5' terminal used for PCR amplification of the IRES fragment

<400> 31
acgcgtcgac cgcccctctc cctccccc 28

<210> 32
<211> 43
<212> DNA
<213> Artificial Sequence

<220>
<223> Designed sequence of a 3'-primer incorporating the Xho I recognition site at the 5' terminal used for PCR amplification of the IRES fragment

<400> 32
ccgctcgaga ttatcatcgt gtttttcaaa ggaaaaccac gtc 43

<210> 33
<211> 61
<212> DNA
<213> Artificial Sequence

<220>
<223> Designed oligonucleotide acting as a sense chain in annealing to construct the coding fragment of the chicken lysozyme secretion signal

<400> 33

ctagaccatg aggtctttgc taatcttggt gctttgcttc ctgcccctgg
ctgctctggg 60
g 61

<210> 34
<211> 57
<212> DNA
<213> Artificial Sequence

<220>
<223> Designed oligonucleotide acting as an anti-sense chain in annealing to construct the coding fragment of the chicken lysozyme secretion signal

<400> 34

ccccagagca gccaggggca ggaagcaaag caccaagatt agcaaagacc
tcatggt 57

<210> 35
<211> 26
<212> DNA
<213> Artificial Sequence

<220>
<223> Designed sequence of a 5'-primer incorporating the Dra I recognition site at the 5' terminal used for PCR amplification of the scFv coding fragment

<400> 35
gcgtttaaag tgacgttgga cgtccg 26

<210> 36
<211> 29
<212> DNA
<213> Artificial Sequence

<220>
<223> Designed sequence of a 3'-primer incorporating the BamH I recognition site at the 5' terminal used for PCR amplification of the scFv coding fragment

<400> 36
attaggatcc gcgcttaagg acggtcagg 29

<210> 37
<211> 35
<212> DNA
<213> Artificial Sequence

<220>
<223> Designed sequence of a 5'-primer incorporating the BamH I recognition site at the 5' terminal used for PCR amplification of the coding fragment of the human antibody heavy chain γ1 Fc region

<400> 37
attaggatcc gagcccaaat cttgtgacaa aactc 35

SEQUENCE LISTING

<110> Kaneka Corporation, Nagoya Industrial Science Research Institute (Chubu Technology Licensing Office)

<120> Method of expressing gene in transgenic birds using retrovirus vector and transgenic birds thus obtained

<130> T753/TRANS-1

<150> JP P2002-236089
<151> 2002-08-13

<160> 37

<210> 1
<211> 25
<212> DNA
<213> Artificial Sequence

<220>
<223> Designed sequence of a 5'-primer used for PCR amplification of the Miw promoter 5' region fragment

<400> 1
cggtctagag gaattcagtg gttcg 25

<210> 2
<211> 26
<212> DNA
<213> Artificial Sequence

<220>
<223> Designed sequence of a 3'-primer incorporating the BamH I recognition site at the 5' terminal used for PCR amplification of the Miw promoter 5' region fragment

<400> 2
ccaggatccg acgttgtaaa acgacg 26

<210> 3
<211> 28
<212> DNA
<213> Artificial Sequence

<220>
<223> Designed sequence of a 5'-primer incorporating the Hind III recognition site at the 5' terminal used for PCR amplification of the Miw promoter 3' region fragment

<400> 3
ccaaagcttg ccgcagccat tgcctttt 28

<210> 4
<211> 27
<212> DNA
<213> Artificial Sequence

<220>
<223> Designed sequence of a 3'-primer incorporating the Bln I recognition site at the 5' terminal used for PCR amplification of the Miw promoter 3' region fragment

<400> 4
atacctaggg gctggctgcg gaggaac 27

<210> 5
<211> 29
<212> DNA
<213> Artificial Sequence

<220>
<223> Designed sequence of a 5'-primer incorporating the Nhe I recognition site at the 5' terminal used for PCR amplification of the chicken β-actin promoter fragment lacking the intron

<400> 5
tttagctagc tgcagctcag tgcatgcac 29

<210> 6
<211> 27
<212> DNA
<213> Artificial Sequence

<220>
<223> Designed sequence of a 3'-primer incorporating the Xba I recognition site at the 5' terminal used for PCR amplification of the chicken β-actin promoter fragment lacking the intron

<400> 6
ataatctaga aacgcagcga ctcccgc 27

<210> 7
<211> 25
<212> DNA
<213> Artificial Sequence

<220>
<223> Designed sequence of a 5'-primer incorporating the Xho I recognition site at the 5' terminal used for PCR amplification of the coding fragment of the human antibody light chain κ constant region

<400> 7
atcctcgaga ggccaaagta cagtg 25

<210> 8
<211> 33
<212> DNA
<213> Artificial Sequence

<220>
<223> Designed sequence of a 3'-primer incorporating the BamH I recognition site at the 5' terminal used for PCR amplification of the coding fragment of the human antibody light chain κ constant region

<400> 8
cccggatccc taacactctc ccctgttgaa gct 33

<210> 9
<211> 48
<212> DNA
<213> Artificial Sequence

<220>
<223> Designed sequence of a 5'-primer incorporating the Not I recognition site at the 5' terminal used for PCR amplification of the coding fragment of the human antibody light chain variable region

<400> 9
agcggccgct acaggtgtcc actccgacat cgtgatgacc cagtctcc 48

<210> 10
<211> 34
<212> DNA
<213> Artificial Sequence

<220>
<223> Designed sequence of a 3'-primer incorporating the Xho I recognition site at the 5' terminal used for PCR amplification of the coding fragment of the human antibody light chain variable region

<400> 10
cctctcgagg atagaagtta ttcagcaggc acac 34

<210> 11
<211> 32
<212> DNA
<213> Artificial Sequence

<220>
<223> Designed sequence of a 5'-primer incorporating the Xho I recognition site at the 5' terminal used for PCR amplification of the coding fragment of the human antibody heavy chain μ constant region

<400> 11
acctcgagcg tggccgttgg ctgcctcgca ca 32

<210> 12
<211> 32
<212> DNA
<213> Artificial Sequence

<220>
<223> Designed sequence of a 3'-primer incorporating the Hind III recognition site at the 5' terminal used for PCR amplification of the coding fragment of the human antibody heavy chain μ constant region

<400> 12
actaagctta cgttgtacag ggtgggttta cc 32

<210> 13
<211> 48
<212> DNA
<213> Artificial Sequence

<220>
<223> Designed sequence of a 5'-primer incorporating the Not I recognition site at the 5' terminal used for PCR amplification of the coding fragment of the human antibody heavy chain variable region

<400> 13
agcggccgct acaggtgtcc actccgaggt gcagctggtg gagtctgg 48

<210> 14
<211> 36
<212> DNA
<213> Artificial Sequence

<220>
<223> Designed sequence of a 3'-primer incorporating the Xho I recognition site at the 5' terminal used for PCR amplification of the coding fragment of the human antibody heavy chain variable region

<400> 14
cacgctcgag gtatccgacg gggaattctc acagga 36

<210> 15
<211> 49
<212> DNA
<213> Artificial Sequence

<220>
<223> Designed sequence of a 5'-primer incorporating the Hind III recognition site at the 5' terminal used for DNA polymerase reaction to construct the coding fragment of the human epidermal growth factor receptor transmembrane region

<400> 15
cccaagcttg atctccactg ggatggtggg ggccctcctc ttgctgctg 49

<210> 16
<211> 78

<212> DNA
<213> Artificial Sequence

<220>
<223> Designed sequence of a 3'-primer incorporating the BamH I recognition site at the 5' terminal used for DNA polymerase reaction to construct the coding fragment of the human epidermal growth factor receptor transmembrane region

<400> 16

cccggatcct cagtcaaggc gccttcgcat gaagaggccg atccccaggg ccaccaccag 60
cagcaagagg agggcccc 78

<210> 17
<211> 31
<212> DNA
<213> Artificial Sequence

<220>
<223> Designed oligonucleotide used for site-directed mutagenesis to generate the Nar I recognition site at the 3' terminal of the coding fragment of the human antibody light chain variable region

<400> 17
tgaagacaga tggcgccgcc acagttcgtt t 31

<210> 18
<211> 30
<212> DNA
<213> Artificial Sequence

<220>
<223> Designed oligonucleotide used for site-directed mutagenesis to generate the BamH I recognition site at the 3' terminal of the coding fragment of the human antibody heavy chain variable region

<400> 18
tggggcggat gcggatcctg aggagacggt 30

<210> 19
<211> 30
<212> DNA
<213> Artificial Sequence

<220>
<223> Designed sequence of a 5'-primer incorporating the Not I recognition site at the 5' terminal used for PCR amplification of the coding fragment of the mouse antibody light chain variable region

<400> 19
cgcggccgcc tcagggaaag tttgaagatg 30

<210> 20
<211> 36
<212> DNA
<213> Artificial Sequence

<220>
<223> Designed sequence of a 3'-primer incorporating the Nar I recognition site at the 5' terminal used for PCR amplification of the coding fragment of the mouse antibody light chain variable region

<400> 20
cggcgccgcc acagtccgtt ttatttccag cttggt 36

<210> 21
<211> 30
<212> DNA
<213> Artificial Sequence

<220>
<223> Designed sequence of a 5'-primer incorporating the Not I recognition site at the 5' terminal used for PCR amplification of the coding fragment of the mouse antibody heavy chain variable region

<400> 21
cgcggccgcg aacacggamc cctcaccatg 30

<210> 22
<211> 28
<212> DNA
<213> Artificial Sequence

<220>
<223> Designed sequence of a 3'-primer incorporating the BamH I recognition site at the 5' terminal used for PCR amplification of the coding fragment of the mouse antibody heavy chain variable region

<400> 22
cggatcctgc agagacagtg accagagt 28

<210> 23
<211> 18
<212> DNA
<213> Artificial Sequence

<220>
<223> Designed sequence of a 5'-primer used for PCR amplification of the coding fragment of the human antibody heavy chain γ1 constant region

<400> 23
caagcttcaa gggcccat 18

<210> 24
<211> 19
<212> DNA
<213> Artificial Sequence

<220>
<223> Designed sequence of a 3'-primer used for PCR amplification of the coding fragment of the human antibody heavy chain γ1 constant region

<400> 24
atttacccgg agacaggga 19

<210> 25
<211> 30
<212> DNA

<213> Artificial Sequence

<220>
<223> Designed sequence of a 5'-primer incorporating the BamH I recognition site at the 5' terminal used for PCR amplification of the coding fragment of the human antibody heavy chain γ1 constant region

<400> 25
ataggatccg ctagcttcaa gggcccatcg 30

<210> 26
<211> 30
<212> DNA
<213> Artificial Sequence

<220>
<223> Designed sequence of a 3'-primer incorporating the Hind III recognition site at the 5' terminal used for PCR amplification of the coding fragment of the human antibody heavy chain γ1 constant or Fc region

<400> 26
agcaagcttt catttacccg gagacaggga 30

<210> 27
<211> 28
<212> DNA
<213> Artificial Sequence

<220>
<223> Designed sequence of a 5'-primer incorporating the Sal I recognition site at the 5' terminal used for PCR amplification of the chicken β-actin promoter fragment lacking the intron

<400> 27
acgcgtcgac gtgcatgcac gctcattg 28

<210> 28
<211> 26
<212> DNA
<213> Artificial Sequence

<220>
<223> Designed sequence of a 3'-primer incorporating the Sal I recognition site at the 5' terminal used for PCR amplification of the chicken β-actin promoter fragment lacking the intron

<400> 28
acgcgtcgac aacgcagcga ctcccg 26

<210> 29
<211> 30
<212> DNA
<213> Artificial Sequence

<220>
<223> Designed sequence of a 5'-primer incorporating the Sal I recognition site at the 5' terminal used for PCR amplification of the coding fragment of the antibody κ light chain

<400> 29
aatgtcgaca tggtgtccac ttctcagctc 30

<210> 30

<211> 30
<212> DNA
<213> Artificial Sequence

<220>
<223> Designed sequence of a 3'-primer incorporating the Sal I recognition site at the 5' terminal used for PCR amplification of the coding fragment of the antibody κ light chain

<400> 30
ttcgtcgacc taacactctc ccctgttgaa 30

<210> 31
<211> 28
<212> DNA
<213> Artificial Sequence

<220>
<223> Designed sequence of a 5'-primer incorporating the Sal I recognition site at the 5' terminal used for PCR amplification of the IRES fragment

<400> 31
acgcgtcgac cgcccctctc cctccccc 28

<210> 32
<211> 43
<212> DNA
<213> Artificial Sequence

<220>
<223> Designed sequence of a 3'-primer incorporating the Xho I recognition site at the 5' terminal used for PCR amplification of the IRES fragment

<400> 32
ccgctcgaga ttatcatcgt gtttttcaaa ggaaaaccac gtc 43

<210> 33
<211> 61
<212> DNA
<213> Artificial Sequence

<220>
<223> Designed oligonucleotide acting as a sense chain in annealing to construct the coding fragment of the chicken lysozyme secretion signal

<400> 33

ctagaccatg aggtctttgc taatcttggt gctttgcttc ctgcccctgg ctgctctggg 60
g 61

<210> 34
<211> 57
<212> DNA
<213> Artificial Sequence

<220>
<223> Designed oligonucleotide acting as an anti-sense chain in annealing to construct the coding fragment of the chicken lysozyme secretion signal

<400> 34

ccccagagca gccaggggca ggaagcaaag caccaagatt agcaaagacc
tcatggt 57

<210> 35
<211> 26
<212> DNA
<213> Artificial Sequence

<220>
<223> Designed sequence of a 5'-primer incorporating the Dra I recognition site at the 5' terminal used for PCR amplification of the scFv coding fragment

<400> 35
gcgtttaaag tgacgttgga cgtccg 26

<210> 36
<211> 29
<212> DNA
<213> Artificial Sequence

<220>
<223> Designed sequence of a 3'-primer incorporating the BamH I recognition site at the 5' terminal used for PCR amplification of the scFv coding fragment

<400> 36
attaggatcc gcgcttaagg acggtcagg 29

<210> 37
<211> 35
<212> DNA
<213> Artificial Sequence

<220>
<223> Designed sequence of a 5'-primer incorporating the BamH I recognition site at the 5' terminal used for PCR amplification of the coding fragment of the human antibody heavy chain γ1 Fc region

<400> 37
attaggatcc gagcccaaat cttgtgacaa aactc 35

## Description

### TECHNICAL FIELD

The present invention relates to a G0 transgenic chimera bird producing an antibody, for example an scFv-Fc antibody, in blood and eggs. Moreover, the invention relates to a production method of an antibody which comprises producing a G0 transgenic, chimera bird introduced an exogenous antibody gene with a replication-defective retrovirus vector, and recovering an antibody produced in blood, albumen, or egg yolk. Furthermore, the present invention also relates to a production method of a G0 transgenic chimera bird efficiently expressing a transgene, and the G0 transgenic chimera bird obtainable by said production method.

### BACKGROUND ART

As a means of research for gene functions, researches on transgenic animals incorporated with an exogenous gene in the host have been actively conducted. These transgenic animals are useful not only in fundamental research, but also in industrial applications such as breed improvement, substance production, and donors for replacement organs. An attempt for causing milk of cows, goats, sheep, etc. to produce a biologically active substance has been approaching to practical use. As typical examples thereof, $\alpha$1-antitrypsin and antithrombin are now in clinical stage objecting for applications in pharmaceutical products.

Poultries, typically quails and chickens, have long been bred for meat and eggs they lay, thus various approaches regarding breed improvement such as disease tolerance and flesh improvements objecting for transgenic research are also presumable. In addition, since birds require short period for reaching sexual maturation and can be raised in small spaces, they are thought as low cost protein expression systems and expected for their transgenic production as a production means of antibody pharmaceutical products and rare protein. Since bird eggs contain a large amount of protein and are laid every day, they are thought to become an efficient production system if a transgenic product can be systematically produced as a recombinant protein in eggs.

As for monoclonal antibodies, which have been placed on market in many items and have attracted attention as pharmaceutical products in recent years, the market diffusion thereof is prevented since they cannot be produced in a cheap expression system such as Escherichia coli, and the unit value thereof is expensive. Meanwhile, bird cells have functions for constituting an antibody protein, and transgenic birds can be expected as a production means of an antibody for medical applications, etc., which have conventionally been difficult to be mass-produced. Furthermore, these protein products are thought to have high possibility for having advantageous characteristics for the applications in medicine and testing agent such as improved stability in blood by being given a sugar chain by bird cells.

As described above, transgenic birds are expected for the applications as production means of useful protein, but on the other hand, regardless of various attempts which have so far been conducted, a case successfully accumulating an intended recombinant protein in a bird egg at a practical level is not found yet. Furthermore, a case successfully producing transgenic birds expressing a protein having a conformation comprising a plurality of units such as an antibody at high concentration is also not found yet.

Harvey et al. (Harvey, A. J et al. (2002) Nature Biotechnology. 19, 396) produced a G0 transgenic chimera chicken using a vector derived from Avian Leukosis Virus and introducing $\beta$-lactamase gene into a chicken, but the amount of enzyme expression to serum or egg was as much as about 50 to 250 ng/ml. G1 to G3 transgenic chimera chickens produced by mating the G0 transgenic chimera chickens increased in the amount of enzyme expression by introducing genes into whole.somatic cells, but the increased level remains as much as about several $\mu$g/ml, thus they are still far from practical application.

Generally, for producing a transgenic animal, a method comprising microinjecting DNA to a pronucleus of a fertile egg is used, but this method cannot be applied to birds. That is because it is difficult to obtain an embryo of one-cell stage, and even if it can be obtained, there is no technology for distinguishing the nucleus in the egg. For obtaining the embryo of one-cell stage, it is necessary to obtain an egg immediately after fertilization from an oviduct of a female bird, and to develop the egg normally. In recent years, Perry established a system for obtaining a hen precleavage cell and culturing the cell outside the body (Perry, M. M (1988) Nature, 331). However, even with this technology, it is impossible to distinguish the nucleus within the egg and to introduce an intended gene into the nucleus.

Therefore, gene introduction to a bird fertile egg has been restricted to DNA injection to cytoplasm, and use of DNA inclusion lipid bilayer (liposome), a calcium phosphate method or an electroporation method have been attempted. However, with these technologies, the gene introduction efficiency is poor, and the possibility of the introduced plasmid DNA being introduced into a chromosome is low. Although the gene introduction method by microinjection can efficiently transfer an intended DNA to a fertile egg, since the introduced plasmid DNA is not incorporated into a host chromosome, the transgene plasmid is omitted accompanied by somatic cell division of the host, thus the stable gene introduction effect cannot be desired.

In 1986, the production example of a transgenic chicken using a retrovirus vector was for the first time reported (Salter, D. W et al. (1986) Poultry Sci., 65). The technology of injecting a retrovirus vector into a fertile egg by the microinjection method is a technology high in gene introduction efficiency, and for the birds to which DNA cannot be directly injected into the nucleus, that is the only one practical method for producing stable transgenic inserted with an intended gene into the chromosome.

The present inventors have made intensive investigations, and as a result, they found a production method of a transgenic bird comprising using a safe replication-defective virus vector which is also applied for gene therapy, and efficiently introducing an intended gene (Japanese Kokai Publication 2002-176880). By this method, it became possible to safely and efficiently produce a transgenic bird having a plurality of transgene copies. Moreover, it was also found that the transgene is transmitted to the next generation at high efficiency by this technology, and the use of a transgenic bird as a substance production system has approached to practical use.

However, since most of the genes introduced at this time is inactivated by the host (called gene silencing) at an early stage of development, the protein production amount as a result of gene expression was very small. Although the biological mechanism of transgene inactivation has not been clarified yet, a technology of preventing this inactivation and efficiently expressing an intended gene is indispensable for the application development of a transgenic bird.

SUMMARY OF THE INVENITON

For specifying the time of the gene, which has been introduced to a fertile egg using a retrovirus vector, being inactivated after the generation, the present inventors studied how the expression amount changes by introducing a vector at various stages after the fertilization using a β-galactosidase expression gene as an indicator. As a result, they found that the expression amount of the transgene significantly changes depending on the time when said gene is introduced during the embryo development. Thus, the invention has been completed. That is, the inactivation of the transgene is remarkable relative to the gene introduced immediately after the spawning, and expression frequency of the gene introduced after the lapse of a certain period of time after the spawning is high.

The present inventors used this notice, and found that if an exogenous gene is introduced to an early embryo after the lapse of specific time from the start of the incubation, which is dependent on the bird species, it becomes possible to efficiently express an intended gene without the influence of the inactivation by a host.

Furthermore, when the inventors produced a vector incorporated with a gene encoding a chimera antibody useful as pharmaceutical products, e.g. an scFv-Fc (single chain antibody), and introduced the vector to a quail to produce a G0 transgenic chimera bird by this method, it was found that an antibody derived from the transgene is expressed at high concentration in blood, albumen and egg yolk.

That is, the first aspect of the present invention relates to
a production method of a G0 transgenic chimera bird
which comprises incubating a bird fertile egg, infecting an early embryo after and exclusive of a blastodermal period immediately after the spawning (stage X) with a replication-defective retrovirus vector, and then hatching the embryo, and a G0 transgenic chimera bird produced by said method.

The second aspect of the present invention relates to
a G0 transgenic chimera bird
which is introduced an exogenous antibody gene with a replication-defective retrovirus vector, and
produces an antibody derived from a transgene in at least one of blood, albumen, and egg yolk.

The third aspect of the present invention relates to
a production method of an antibody
which comprises producing the above G0 transgenic chimera bird, and recovering the antibody from blood and/or an egg of said G0 transgenic chimera bird.

It was confirmed that the expression of a transgene by the G0 transgenic chimera bird of the present invention is maintained even when the G0 transgenic chimera bird grows to the adult bird. Therefore, by the invention, it becomes possible to construct a practical production system which comprises introducing a specific gene into a bird, and producing an intended protein even after growing. Moreover, the transgene of the G0 transgenic chimera bird produced by the invention is transmitted to the next generation at high transmission efficiency by mating. Since the transgene is transmitted to the next generation in the form incorporated in the chromosome, the production system by a transgenic bird can provide stable substance production.

By the invention, the intended protein produced in a somatic cell of the transgenic bird is secreted in blood, and can be used by the separation from serum.

Furthermore, the inventors also found that an antibody having the constant region belonging to class human IgG and an antibody having the constant region of quail IgG, chicken IgG or mouse IgG are efficiently transmitted from blood to eggs in quails and chickens. When the antibody having these constant regions is expressed in the G0 transgenic chimera bird produced by the method of the invention, the antibody secreted in blood is accumulated at high concentration in eggs.

When the intended protein is acquired by introducing a gene to transgenic animals, in mammals, the protein is generally secreted in milk and recovered, but in a transgenic bird, an object is accumulated in eggs, and recovered and purified from albumen and egg yolk thereof.

In hen's egg and the like, 30% of the whole component is a protein, and the protein occurring as the main component is ovalbumin. Conventionally, as a method for expressing a recombinant protein in transgenic bird eggs, a view of using an expression promoter of ovalbumin and the like, incorporating a gene coding for an intended protein in the downstream, and expressing an object in albumen in lieu of ovalbumin has been taken.

However, in the method of the invention, it becomes possible to cause secretion of an antibody expressed in a large amount e.g. said IgG class antibody, etc. under the control of a constitutive promoter, e.g. chicken β-actin promoter, etc. in blood to accumulate thereof in eggs. In this description, the constitutive promoter refers to a promoter systemically expressed.

In addition, for specifying the essential part for transition into eggs among the antibody structures, modified antibodies, Fab and Fc fragments, were inoculated into quail and chicken blood to test the transition ability into eggs. As a result, Fc fragment was accumulated in eggs, and it was suggested that the antibody transition into eggs are carried out through Fc receptor.

From that notice, as a method for universally using the production method of an antibody by a transgenic bird in eggs as a general production method of protein, presumable is a production method comprising designing a vector producing a protein having the structure of human IgG constant region (Fc) being fused, producing a transgenic bird, recovering a protein containing an object from eggs, and cutting the Fc portion to purifying the object.

Furthermore, conventionally, when an antibody was produced using a mammalian transgenic animal, the problem of difficulty in separating and purifying the autoantibody occurring in the production animal and an intended antibody has been pointed out. As an advantage of the antibody production method using a bird as the production animal, there may be mentioned an easiness of separation of the autoantibody of the bird and intended recombinant antibody since the autoantibody is not adsorbed on protein A and protein G columns.

As mentioned above, although a human monoclonal antibody is useful as a pharmaceutical product, there is only an expensive production means which use an animal cell culture or mouse ascitic fluid, and its high unit value prevents market diffusion thereof.

In recent years, an scFv (single chain antibody) in which only V regions of H chain and L chain of the antibody are bonded with a linker sequence by gene engineering technique is produced, and this has an advantage of being producible by Escherichia coli so that attracts attention in view of costs. However, these proteins called low molecular antibodies are low in stability in blood, thus are difficult in the practical utility as therapeutic and testing uses.

Although an scFv-Fc, which is producible by bonding an Fc region to an scFv, is stable also in blood and is considered to be more practical, it cannot be produced by Escherichia coli, and can be supplied only by a bioreactor using an animal cell. A humanized scFv-Fc, which is producible by bonding a human Fc to the scFv having a binding region produced by other animal such as chicken, has promise also for therapeutic uses. When this protein can be mass-produced by the G0 transgenic chimera bird of the invention, its availability is high.

The G0 transgenic chimera bird of the invention can be applied to the mass-production of the antibody protein that can be produced only in a small amount by the conventional manner, i.e. a recombinant antibody such as an scFv-Fc, chimera antibody, human monoclonal antibody, etc. at low cost, and to use thereof by practically recovering and purifying.

Thus, in the present invention, a production method of efficiently expressing a transgene in the G0 transgenic chimera bird using a retrovirus vector is disclosed. Also disclosed in the invention is a production method of a G0 transgenic chimera bird producing an intended useful protein in bird somatic cells by introducing a specific gene. Further disclosed in the invention is a protein production system low in the production cost which comprises causing bird cells to produce substances useful as pharmaceutical and testing products such as a monochronal human type antibody, chimera antibody, scFv-Fc antibody, and functional protein having a complicated confirmation, which cannot be conventionally produced by Escherichia coli, etc., and utilizing thereof by recovering from serum and blood.

Furthermore, it is also presumable to provide a technology of modifying domestic fowls in a preferable way by applying the production method of a G0 transgenic chimera bird of the present invention and a modified bird. As the characteristics of domestic birds, improved flesh quality, improved disease resistance, improved growth rate, and the like are preferred. Birds also have various demands for pets, and the production method of the invention can also be applied as a means for breed improvement to preferable characteristics for pets such as improvement in feather colors and decrease in aggressiveness in a short period.

Furthermore, usually, transgenic production of mammals, amphibians, fishes, and the like is currently carried out by a nuclear transfer, but the invention is also applicable as a new alternative efficient technology of transgenic animal production.

That is, the present invention relates to a G0 transgenic chimera bird which is introduced an exogenous antibody gene with a replication-defective retrovirus vector, and produces an antibody derived from a transgene in at least one of blood, albumen, and egg yolk. The constant region of the above antibody is preferably a class belonging to human IgG, subclass belonging to human IgG1, quail IgG, chicken IgG, or mouse IgG. The above antibody gene is preferably controlled by a constitutive promoter, and the above constitutive promoter is preferably chicken β-actin promoter. The above retrovirus vector is preferably a vector derived from Moloney murine leukemia virus, and a VSV-G pseudo type one is preferred. The G0 transgenic chimera bird of the invention is preferably a chicken or quail. The above antibody is preferably a chimera antibody, and the production amount of the above chimera antibody is preferably not less than 0.5 μg/ml, more preferably not less than 5 μg/ml in blood. In albumen, it is preferably not less than 0.1 μg/ml, more preferably not less than 1 μg/ml, and in egg yolk, it is preferably not less than 0.1 μg/ml, more preferably not less than 1 μg/ml. The above antibody is preferably an scFv-Fc antibody, and the production amount of said scFv-Fc antibody is preferably not less than 20 μg/ml, more preferably not less than 2000 μg/ml in blood. In albumen, it is preferably not less than 5 μg/ml, more preferably not less than 500 μg/ml, and in egg yolk, it is preferably not less than 5 μg/ml, more preferably not less than 500 μg/ml. A production method of an antibody comprising producing the G0 transgenic chimera bird of the invention, and recovering the antibody from blood and/or eggs of said G0 transgenic chimera bird is also one aspect of the invention.

A production method of a G0 transgenic chimera bird which comprises incubating a bird fertile egg, infecting an early embryo after and exclusive of a blastodermal period immediately after the spawning with a replication-defective retrovirus vector, and then hatching the embryo is also one aspect of the invention. In the production method of a G0 transgenic chimera bird of the invention, the time of infecting a replication-defective retrovirus vector is preferably after 24 hours or more from the start of the incubation. The method of infecting a replication-defective retrovirus us vector preferably comprises an early embryo microinjected the vector into a heart or blood vessel formed in an early embryo. The above heart or blood vessel is preferably formed in the early embryo after 24 hours or more from the start of the incubation. As for the activity of the replication-defective retrovirus vector to be microinjected, the vector preferably has the titer of not less than 1 x $10^7$ cfu/ml, more preferably not less than 1 x $10^8$ cfu/ml, and still more preferably not less than 1 x $10^9$ cfu/ml. The above retrovirus vector is preferably a vector derived from from Moloney murine leukemia virus, and a VSV-G pseudo type one is preferred. The G0 transgenic chimera bird produced by the invention is preferably a chicken or quail. The transgene incorporated into the above replication-defective retrovirus vector preferably contains a gene sequence not derived from a retrovirus. The above gene sequence not derived from a retrovirus is preferably a gene sequence controlled by chicken β-actin promoter, and is preferably a gene sequence coding for an antibody gene or fusion protein gene. The above antibody gene is preferably a chimera antibody gene, and an scFv-Fc antibody gene is preferred.

The G0 transgenic chimera bird produced by the production method of a G0 transgenic chimera bird of the invention is also one aspect of the present invention.

BRIEF DESCRIPTION OF THE DRAWINGS

Fig. 1 shows the structure of vector construct pMSCVNΔAβ of a replication-defective retrovirus vector. $Neo^r$ represents a neomycin resistance gene and $Amp^r$ represents an ampicillin resistance gene. $P_{\Delta Act}$ represents a β-actin promoter gene. β-Gal represents a β-galactosidase expression gene. Ψ+ represents a packaging signal sequence. 5'LTR and 3'LTR each represents a long terminal repeat sequence of MoMLV.

Fig. 2 shows the relation between the introduction time of a gene and β-galactosidase activity expression in a G0 transgenic chimera quail. The horizontal axis shows incubation time (hr), and the vertical axis shows the β-galactosidase activity expressed in mUnit/mg.

Fig. 3 shows the relation between the introduction time of a gene and the β-galactosidase activity expression in a G0 transgenic chimera chicken. The horizontal axis shows incubation time (hr), and the vertical axis shows the β-galactosidase activity expressed in mUnit/mg.

Fig. 4 shows the relation between the titer of the introduced retrovirus vector, and the β-galactosidase activity expression in a G0 transgenic chimera quail. The horizontal axis shows the virus titer expressed in cfu/ml, and the vertical axis shows the β-galactosidase activity expressed in mUnit/mg.

Fig. 5 shows the relation between the titer of the introduced retrovirus vector, and the β-galactosidase activity expression in a G0 transgenic chimera quail. The horizontal axis shows the virus titer expressed in cfu/ml, and the vertical axis shows β-galactosidase activity expressed in mUnit/mg.

Fig. 6 shows human IgG antibody accumulated in quail eggs. The antibody value shows the average value of the same experiment result carried out with three quails.

Fig. 7 shows human IgG antibody accumulated in chicken eggs. The antibody value shows the average value of the same experiment result carried out with three chickens.

Fig. 8 shows Fab fragment accumulated in quail eggs. The antibody value shows the average value of the same experiment result carried out with three quails.

Fig. 9 shows Fab fragment accumulated in chicken eggs. The antibody value shows the average value of the same experiment result carried out with three chickens.

Fig. 10 shows Fc fragment accumulated in quail eggs. The antibody value shows the average value of the same experiment result carried out with three quails.

Fig. 11 shows Fc fragment accumulated in chicken eggs. The antibody value shows the average value of the same experiment result carried out with three chickens.

Fig. 12 shows the structures of anti-CD2 antibody expression vector constructs pMSCV/GΔAL (Fig. 12(A)), pMSCV/GΔAH (Fig. 12(B)), and pMSCV/GΔALIH (Fig. 12(C)). $Amp^r$ represents an ampicillin resistance gene. $P_{\Delta Act}$ represents a β-actin promoter gene. Ψ+ represents a packaging signal sequence. GFP represents a green fuluorescent protein gene. L represents an anti-CD2 antibody light chain gene. H represents an anti-CD2 antibody heavy chain gene. 5'LTR and 3'LTR each represents a long terminal repeat sequence of MoMLV.

Fig. 13 shows the structure of scFv-Fc antibody expression vector construct pMSCV/GΔAscFv-Fc. $Amp^r$ represents an ampicillin resistance gene. $P_{\Delta Act}$ represents a β-actin promoter gene. Ψ+ represents a packaging signal sequence. GFP represents a green fuluorescent protein gene. scFv-Fc represents an scFv-Fc antibody gene. 5'LTR and 3'LTR each represents a long terminal repeat sequence of MoMLV.

Fig. 14 shows the amount of scFv-Fc expressed in G0 transgenic chimera quail serum. The horizontal axis shows the individual number, and the vertical axis shows the concentration of an scFv-Fc antibody (μg/ml).

Fig. 15 shows the amount of an scFv-Fc expressed in G0 transgenic chimera quail eggs. The horizontal axis shows the date of egg collection from the start of spawning, and the vertical axis shows the concentration of an scFv-Fc antibody (μg/ml).

Fig. 16 shows the analysis result of purified scFv-Fc by SDS-PAGE. The lane 1 shows low molecular-weight marker (LMW), and the lane 4 shows high molecular-weight marker (HMW). The lane 2 and lane 3 show the electrophoresis results of an scFv-Fc subjected to reduction treatment and scFv-Fc which is not subjected to reduction treatment, respectively.

DETAILED DESCRIPTION OF THE INVENTION

Hereinafter, the present invention is described in detail.

The G0 transgenic chimera bird of the invention is the bird introduced an exogenous antibody gene with a replication-defective retrovirus vector, and produces an antibody derived from a transgene in blood, albumen, or egg yolk.

The bird to be used in the practice of the invention is not particularly restricted, and for example, there may be mentioned domestic fowls domesticated for eating, spawning and the like purpose such as a chicken, turkey, duck, ostrich and quail, and pet bird. Among them, a chicken and quail are preferred in view of easy availability and fertility as egg production species.

As the retrovirus vector to be used in the invention, there may be mentioned vectors derived from Moloney murine leukemia virus (MoMLV), Avian leukosis virus (ALV), and the like. Among them, those derived from MoMLV are preferred, but the invention is not limited to these.

In consideration of safety, the virus generally used as a transgenic vector is a self replication-defective virus producible by deleting either or whole of three species of genes gag, pol and env which are necessary for replication of virus particles. For efficiently infecting a bird cell with this virus vector, virus vectors obtainable by artificially converting a coat protein to a VSV-G (vesicular stomatitis virus origin) pseudo type one are preferred, but the invention is not limited to this virus type.

Pseudo type virus vectors prepared using a packaging cell, helper virus or the like are introduced into an early embryo, blood vessel, and heart by the general microinjection method (Bosselman R. A et al. (1989) Science 243, 533). As the gene introduction method, in addition to that, lipofection, electroporation and the like methods can be mentioned.

The gene to be introduced into the bird in the practice of the invention is not particularly restricted, but is constituted with a marker gene, a structural gene for expressing an intended protein, a promoter gene controlling expression of these genes, a secretory signal gene, and the like.

As the above marker gene, there may be mentioned a neomycin resistance gene, β-galactosidase gene, LacZ gene, and a gene coding for a fluorescence protein such as GFP (green fluororescent protein).

The above structural gene for expressing an intended protein is not particularly restricted, and there may be mentioned a gene coding for an antibody or an enzyme, etc. useful in the filed of gene industry such as a human monoclonal antibody, and the like. Also usable are genes of other useful biologically active substances. Particularly preferred are structural genes of exogenous antibodies such as an antibody gene having the constant region belonging to class human IgG, antibody gene having the constant region belonging to subclass human IgG1, antibody gene having the constant region of quail IgG, chicken IgG, or mouse IgG in view of their preferable accumulation in eggs.

Furthermore, preferred as the above structural gene is a structural gene of a chimera antibody.
The chimera antibody refers to an antibody constituted of two or more different species of genetic characteristics.
Conventionally, medical antibodies produced by mouse hybridoma are derived from mice, thus there has been a problem that the rejection occurs by the immune system when administered to human bodies. As the above chimera antibody, there may be mentioned, for example, chimera antibodies in which said defect is improved by substituting the regions other than those binding with an antigen protein among mouse antibodies with human antibodies to cause no rejection, such as an anti-human CD2 antibody, anti-CD20 receptor antibody and anti-TNF antibody, and some of them have already been placed on market as pharmaceutical products.
Still more preferred as the above structural gene is a structural gene of an scFv-Fc antibody.
Among medical recombinant antibodies, there is a group called "low molecular antibody". In immunoglobulin IgG, there is a domain comprising hetero dimmers of VH and VL called Fv: Fragment of variable region directly binding with an antigen. The Fv domain alone has a sufficient antigen binding ability even though it has only about 1/5 molecular weight relative to IgG. The one obtainable by artificially binding between the VH and VL domains with a peptide linker is a low molecular antibody called scFv: single chain Fv, and has been known to have improved stability as compared with VH and VL alone.
Powers et al. (Powers, D.B et al. (2000) J Immunol Method. 251, 123) have found that by fusing an Fc portion derived from human IgG1 to the scFv, the stability in blood is increased. This scFv-Fc antibody is thought to be useful for medical applications, but is not produced by Escherichia coli, which is a low-cost mass production system.
As other preferable gene sequence mentioned above, there may be mentioned a structural gene of a fusion protein.
A group of artificial proteins in which parts of two or more species of proteins are fused by gene recombination is called a fusion protein. Among those already have been put into practical use as pharmaceutical products, there are TNFR-Fc prepared by fusing Fc of immunoglobulin to a TNF receptor, LFA3-Fc prepared by fusing Fc to LFA3, or the like. These are artificial proteins designed to have stronger biological activity by fusing Fc to be solubilized.
In the G0 transgenic chimera bird of the invention, by using the human monoclonal antibody gene, chimera antibody gene, and scFv-Fc antibody gene mentioned above as the gene to be introduced into the bird, antibody pharmaceutical products which have conventionally been difficult to produce can be mass-produced at low cost.
For example, in the case of the G0 transgenic chimera bird introduced a chimera antibody gene, the antibody content in blood is preferably not less than 0.5 μg/ml, more preferably not less than 5 μg/ml. In albumen, it is preferably not less than 0.1 μg/ml, more preferably not less than 1 μg/ml, and in egg yolk, it is preferably not less than 0.1 μg/ml, more preferably not less than 1 μg/ml.
Moreover, in the case of the G0 transgenic chimera bird introduced an scFv-Fc antibody gene, the antibody content in blood is preferably not less than 20 μg/ml, more preferably not less than 2000 μg/ml. In albumen, it is preferably not less than 5 μg/ml, more preferably not less than 500 μg/ml, and in egg yolk, it is preferably not less than 5 μg/ml, more preferably not less than 500 μg/ml.
As the above-mentioned promoter gene, there may be mentioned a constitutive promoter. It is preferable when the antibody gene is controlled by the constitutive promoter since the antibody gene expression is stabilized. As more preferable constitutive promoter, there may be mentioned chicken β-actin promoter.
The method of producing an antibody of the invention comprises producing the G0 transgenic chimera bird of the invention, and recovering an antibody from blood and/or egg of the above G0 transgenic chimera bird.
Next, the method of producing the G0 transgenic chimera bird of the invention is described.
As one of said production method, there may be mentioned one which comprises incubating a bird fertile egg, infecting an early embryo after and exclusive of a blastodermal period immediately after the spawning with a replication-defective retrovirus vector, and then hatching the embryo. Furthermore, there may also be mentioned a method as one production method of the invention which comprises incubating a bird fertile egg, infecting an early embryo after the lapse of 24 hours or more from the start of the incubation with a replication-defective retrovirus vector, and then hatching the embryo.
More preferred is a method comprising an early embryo microinjected with a replication-defective retrovirus vector to a heart or blood vessel formed in the early embryo.
That is, the method of producing the G0 transgenic chimera bird of the invention comprises microinjected with a replication-defective retrovirus vector (a fertile egg after the lapse of specific time from spawning). Taking a chicken as an example for the early development of a fertile egg after spawning, firstly, the fertile egg fertilized within an oviduct starts cleavage after about 1.5 hours from the fertilization. The egg in which discoidal cleavage is started with the state of cytoplasm being connected is cleaved for 1 day before released to outside the body, and becomes an embryo called blastoderm consisting of about 60,000 cells (blastoderm period). This blastoderm is observed as a white ring with a diameter of 3 to 4 mm in the center of egg yolk. This embryo is divided into upper and lower layers to form a blastocele. The spawning occurs at about the time a hypoblast is formed. The primitive streak is formed, the blastoderm becomes to have triple structure i.e. upper, middle and lower layers, and then triploplast is formed. Thereafter, an embryo is formed and grown, and hatched on the 22nd day from the ovulation. The blastoderm period is also called a stage X. Since a productive cell generates from a part of the cell of this stage, the fertile egg of this period is conventionally used as a target of gene introduction.

In the practice of the invention, the time when a fertile egg in a blastoderm period immediately after spawning is placed under the environmental condition suited for hatching, for example, in the case of chicken, temperature of 37.7 to 37.8°C and humidity of 50 to 70%, is set as 0 hour, which was set as the start of incubation, and various treatments were conducted with time lapses. The formation of a blood vessel system was observed on egg yolk after 36 hours from the start of incubation in quails, and about after 50 hours in chickens, and pulsation of the organ which is to be differentiated to a heart was observed.

For hatching the fertile egg introduced the above gene, the method comprising using an artificial eggshell developed by the present inventors (Kamihira, M. et al. (1998) Develop. Growth Differ., 40, 449) and the like can be applied.

As the replication-defective retrovirus vector, gene to be introduced, and transgenic bird, which are used in the production method of the invention, there may be mentioned the same ones as of the G0 transgenic chimera bird mentioned above.

The transgene incorporated to the above replication-defective retrovirus vector preferably contains a gene sequence not derived form the retrovirus. Herein, in the production method of the invention, as the "gene not derived form the retrovirus", there may be mentioned the above structural gene, promoter gene, secretion signal gene, and the like. The above gene sequence not derived form retrovirus is preferably a gene sequence controlled by chicken β-actin promoter, and a gene sequence coding for an antibody gene or a fusion protein is preferred.

In the production method of the invention, for microinjection a replication-defective retrovirus vector having titers of not less than 1 x $10^7$ cfu/ml, preferably not less than 1 x $10^8$ cfu/ml, more preferably not less than 1 x $10^9$cfu/ml is preferable in view of the efficient gene introduction.

The bird introduced the gene into the fertile egg by the production method of the invention grows as the transgenic bird having a transgene in its somatic cell in mosaic shape. The first generation transgenic bird is called the G0 transgenic chimera bird.

Such G0 transgenic chimera bird obtained by the production method of the invention is also one aspect of the invention.

When the second and third generation bird born by mating the G0 transgenic chimera bird and a nontransgenic bird, or the G0 transgenic chimera bird each other is generated from a productive cell having a transgene in the chromosome, the born bird grows up as an individual containing the transgene within the somatic cells of the entire body. The offspring inheriting the transgene from the individual of the G0 transgenic chimera bird are called G1, G2, G3 transgenic birds through generations.

By mating the G0 transgenic chimera bird of the invention with an allogeanic nontransgenic bird or mating type G0 transgenic chimera bird, the transgene can be transmitted to offspring, and also a complete transgenic bird having the transgene in somatic cells of the entire body can be produced. Since the complete transgenic bird has somatic cells having a transgene at high ratio, it can be expected that the production amount of recombinant protein derived from a transgene is increased as compared with the G0 transgenic chimera bird. Furthermore, by establishing the line of transgenic bird which stably transmits the transgene, it becomes possible to stabilize the quality as a protein production system.

## BEST MODE FOR CARRYING OUT THE INVENTION

In the following, the present invention is described in detail by means of examples, however, these examples are no limitative of the scope of the invention.

### (Example 1) Preparation of a β-galactosidase expression vector construct

β-galactosidase expression vector construct pMSCVNΔAβ was produced as follows.

1. A Rous sarcoma virus (RSV) promoter fragment was cut from plasmid pLXRN (product of BD Biosciences Clontech) using restriction enzymes XhoI and HindIII, and then inserted into XhoI and HindIII sites of plasmid pBluescriptIISK (+) (product of Stratagene) to produce plasmid pBlue/RSV.

2. A β-galactosidase (β-Gal) gene fragment was cut from plasmid pCMVβ (product of BD Biosciences Clontech) using restriction enzyme NotI, and then inserted into NotI site of plasmid pZeoSV2 (+) (product of Invitrogen Corporation). The plasmid having the structure of a β-Gal gene being inserted in the same direction as T7 promoter was named pZeo/lacZ.

3. An RSV promoter fragment was cut from pBlue/RSV using restriction enzymes XhoI and PstI. A β-Gal gene fragment was cut from pZeo/lacZ using restriction enzymes PstI and XhoI. A vector fragment of plasmid pLNHX (product of BD Biosciences Clontech) treated with restriction enzyme XhoI was linked to the above two cut fragments to produce plasmid pLNRβ.

4. A fragment containing a series of Moloney murine sarcoma virus (MoMuSV) 5'-long terminal repeat (LTR), virus packaging signal, and a neomycin resistance ($Neo^r$) gene was cut from pLNHX using restriction enzymes SacII and XhoI, and then linked to a vector fragment of pLXRN treated with restriction enzymes SacII and XhoI to produce plasmid pLXL.

5. A β-Gal gene fragment was cut from pZeo/lacZ using restriction enzymes HindIII and XhoI, and then linked to a vector fragment of pLXL treated with restriction enzymes HindIII and XhoI to produce plasmid pLZL.

6. By PCR (94°C/15 seconds, 55°C/30 seconds, 72°C/1.5 minutes: 35 cycles; KOD-Plus-DNA polymerase (product of Toyobo Co., Ltd.)) using two chemosynthesis oligonucleotides 5'-cggtctagaggaattcagtggttcg-3' (SEQ ID NO:1) and 5'-ccaggatccgacgttgtaaaacgacg-3' (SEQ ID NO:2; underlined portion is BamHI restriction enzyme site) as primers, 5' region fragment of a hybrid promoter (Miw promoter) of RSV promoter and chicken β actin (Act) promoter were amplified from plasmid pMiwZ (Suemori et al., 1990, Cell Diff. Dev. 29: 181 to 185), cut with restriction enzymes BamHI and MunI, and then inserted into BamHI and MunI sites of plasmid pGREEN LANTERN-1 (product of Gibco BRL) to produce plasmid pGmiw5'.

7. A Miw promoter 5'-side central region fragment was cut from pMiwZ using restriction enzymes MunI and ClaI, and then inserted into MunI and ClaI sites of pGmiw5' to produce plasmid pGmiw5'-2.

8. A fragment containing Miw promoter 5' region and 5'-side central region was cut from pGmiw5'-2 using restriction enzymes BamHI and EcoRI, and then inserted into BamHI and EcoRI sites of pBluescript IISK (+) to produce plasmid pBlue/Miw5'.

9. By PCR (98°C/15 seconds, 60°C/30 seconds, 72°C/30 seconds: 35 cycles) using two chemosynthesis oligonucleotides 5'-cca<u>aagctt</u>gccgcagccattgcctttt-3' (SEQ ID NO:3; underlined portion is HindIII restriction enzyme site) and 5'-ata<u>cctagg</u>gctggctgcggaggaac-3' (SEQ ID NO:4; underlined portion is BlnI restriction enzyme site) as primers, a Miw promoter 3' region fragment was amplified from pMiwZ, cut with restriction enzymes HindIII and BlnI, and then linked to a vector fragment of pLXL treated with restriction enzymes HindIII and BlnI to produce plasmid pLMiw3'.

10. A Miw promoter 3'-side central region fragment was cut from pMiwZ using restriction enzymes EcoRI and MboII. A Miw promoter 3' region fragment was cut from pLMiw3' using restriction enzymes MboII and KpnI. The above two cut fragments were inserted into EcoRI and KpnI sites of pBlue/Miw5' to produce plasmid pBlue/Miw.

11. A fragment containing full length of Miw promoter was cut from pBlue/Miw using restriction enzymes BamHI and BlnI, and then linked to a vector fragment of pLXL treated with restriction enzymes BamHI and BlnI to produce plasmid pLML.

12. An Act promoter fragment was cut from pLML using restriction enzymes SmaI and XbaI, and then inserted into EcoRV and XbaI sites of pBluescript IISK (+) to produce plasmid pBlue/Act.

13. A Miw promoter fragment was cut from pLML using restriction enzymes HindIII and BglII, and then linked to a vector fragment of pLZL treated with restriction enzymes HindIII and BamHI to produce plasmid pLMβL.

14. An Act promoter fragment was cut from pBlue/Act using restriction enzymes SalI and BlnI. A β-Gal gene fragment was cut from pLMβL using restriction enzymes BlnI and BglII. The above two cut fragments were linked to a vector fragment of pLNRβ treated with restriction enzymes XhoI and BglII to produce plasmid pLNAβ.

15. By PCR (98°C/15 seconds, 60°C/30 seconds, 68°C/2 minutes: 30 cycles) using two chemosynthesis oligonucleotides 5'-ttta<u>gctagc</u>tgcagctcagtgcatgcac-3' (SEQ ID NO:5; underlined portion is NheI restriction enzyme site) and 5'-ataa<u>tctaga</u>aacgcagcgactcccgc-3' (SEQ ID NO:6; underlined portion is XbaI restriction enzyme site) as primers, an intron-defective actin (ΔAct) promoter fragment was amplified from pMiwZ, and then a fragment containing a part of ΔAct promoter was cut using restriction enzymes XhoI (XhoI restriction enzyme site occurs in an amplification fragment) and XbaI. A fragment containing a remaining portion of ΔAct promoter and β-Gal gene was cut from pLNAβ using restriction enzymes BlnI and BglII. The above two cut fragments were linked to a vector fragment of pLNAβ treated with restriction enzymes XbaI and BglII to produce plasmid pLNΔAβ.

16. A fragment containing a series of Neo$^r$ genes, ΔAct promoter, and β-Gal gene was cut from pLNΔAβ using restriction enzymes BlnI and BglII, and then linked to a vector fragment of pLXL treated with restriction enzymes BlnI and BglII to produce plasmid pLNΔAβ-2.

17. A fragment containing a series of Neo$^r$ genes, ΔAct promoter, and β-Gal gene was cut from pLNΔAβ-2 using restriction enzymes BamHI and BglII, and then linked to a vector fragment of plasmid pMSCVneo (product of BD Biosciences Clontech) treated with restriction enzymes BamHI and BglII. The plasmid in which BamHI and BglII sites disappeared was named pMSCVNΔAβ.

The structure of vector construct pMSCVNΔAβ of the thus produced replication-defective retrovirus vector was shown in Fig. 1.

(Example 2) Preparation of a β-galactosidase expression retrovirus vector

In order to prepare a retrovirus vector from the vector construct pMSCVNΔAβ produced in Example 1, 5 x $10^6$ packaging cell GP293 (product of BD Biosciences Clontech) was sown in a 100 mm-diameter culture dish and cultured. The culture medium was changed for a flesh DMEM (Dulbecco's Modified Eagle's Medium), and 8 μg of p-VSV-G vector (product of BD Biosciences Clontech) and 8 μg of pMSCVNΔAβ were introduced into the above GP293 cells by the lipofection method. After the lapse of 48 hours, the culture supernatant containing virus particles were recovered and filtered through a 0.45 μm accetylcellulose filter (Advantech Co., Ltd.) to remove impurities. The obtained solution was added with polybrene (product of Sigma Corporation) so as to be 10 μg/ml to prepare a virus solution.

The prepared virus solution was added to GP293 cells cultured separately, and after the culture for 48 hours, the cells were successively cultured in a culture containing 600 μg/ml of G418 (product of GIBCO BRL) to obtain G418 stably transformed GP293 strain.

The obtained stably transformed strain was cultured in a 10 mm-diameter dish so as to be 80% confluent, and 16 μg of pVSV-G vector was introduced thereto by the lipofection method. After the lapse of 48 hours, 12 ml of the culture supernatant containing virus particles was recovered.

This culture supernatant was subjected to centrifugation at 50,000 g and 4°C for 1.5 hours to precipitate virus. After removal of the supernatant, 50 μl of 50 mM Tris-HCl (pH 7.8), 130 mM NaCl and 1 mM EDTA solution were added to the precipitate containing the virus particles. Then, the mixture was allowed to stand at 4°C overnight and suspended thoroughly to recover a virus solution. The thus obtained high titer virus vector was $10^8$ to $10^9$ cfu/ml.

The virus titer was measured as follows. The day before the measurement, 7 x $10^4$ NIH3T3 cells (purchased from American Type Culture Collection) were sown in a 35 mm-diameter dish and cultured. The virus solution diluted to $10^2$- to $10^6$-fold was added to each dish in 1 ml. After the lapse of 48 hours, the rate of cells expressing GFP (green fluorescent protein) was determined by a fluorescence microscope, and the titer was determined by the following calculation formula.

Virus titer = (number of cells) x (dilution rate) x (expression ratio) (cfu/ml)

(Example 3) Injection of the retrovirus vector to a quail embryo

A quail fertile egg of WE lineage (Japan Bio Science Laboratory CO., Ltd.) was used. At the time when this fertile egg was placed in an incubator with a built-in automatic egg rotation device (product of Showafuranki Co., Ltd.; P-008 type) at 37.9°C and humidity of 65% was set as the incubation start time (0 hour). Then, the incubation was carried out while rotating the egg at 90 degrees every 15 minutes.

At the start of the incubation, the fertile eggshell was sterilized with 70% ethanol, and the sharp-round end portion was cut with a diamond cutter (MINIMO 7C710, product of Minitor Co., Ltd.) in a 2 cm-diameter circle to expose the embryo. While observing the blastoderm with a stereoscopic microscope, a needle prepared by folding the end so as to have a diameter of about 20 μm from a glass tube (CD-1, product of Olympus Corporation) using a micropipette puller (PC-10, product of Olympus Corporation) was stuck, and about 2 μl of the virus solution prepared in Example 2 was microinjected at the center of the blastodermal cavity using a microinjector (Transjector 5246, product of Eppendorf, Co., Ltd.). After filling albumen up to the cut edge of this eggshell, Teflon film (MilliWrap, product of Millipore Corporation) and polyvinylidene chloride wrap (Saran Wrap, product of Asahi Kasei Corporation) were used to cover the egg using albumen as a paste. Then, the incubation was carried out while rotating the egg at 90 degrees every 15 minutes.

After the lapse of 12 and 24 hours from the start of the incubation, virus was injected to the fertile egg in the same manner. After about 36 hours from the start of incubation, the generation of a blood vessel was confirmed on the egg yolk surface; a part thereof pulsed, thus it was observed that a part thereof becomes a heart field with a stereoscopic microscope. After the lapse of 36, 48 and 55 hours from the start of incubation, 2 μl of the virus solution prepared in Example 2 was microinjected to the heart using a microinjector.

(Example 4) Injection of the retrovirus vector to a chicken embryo

A chicken fertile egg (Japan Bio Science Laboratory CO., Ltd.) was used. At the time when this fertile egg was placed in an incubator with a built-in automatic egg rotation device (product of Showafuranki Co., Ltd.; P-008 type) at 37.9°C and humidity of 65% was set as the incubation start time (0 hour). Then, the incubation was carried out while rotating the egg at 90 degrees every 15 minutes.

At the start of the incubation, the fertile eggshell was sterilized with 70% ethanol, and the sharp-round end portion was cut with a diamond cutter (MINIMO 7C710, product of Minitor Co., Ltd.) in a 3.5 cm-diameter circle to expose the embryo. While observing the blastoderm with a stereoscopic microscope, a needle prepared by folding the end so as to have a diameter of about 20 μm from a glass tube (CD-1, product of Olympus Corporation) using a micropipette puller (PC-10, product of Olympus Corporation) was stuck, and about 2 μl of the virus solution prepared in Example 2 was microinjected at the center of the blastodermal cavity using a microinjector (Transjector 5246, product of Eppendorf, Co., Ltd.). After filling albumen up to the cut edge of this eggshell, Teflon film (MilliWrap, product of Millipore Corporation) and polyvinylidene chloride wrap (Saran Wrap, product of Asahi Kasei Corporation) were used to cover the egg using albumen as a paste. Then, the incubation was carried out while rotating the egg at 90 degrees every 15 minutes.

After the lapse of 12 and 24 hours from the start of the incubation, the fertile egg was treated in the same manner. After about 50 hours from the start of incubation, the generation of a blood vessel was observed on the egg yolk surface; a part thereof pulsed, thus it was observed that a part thereof becomes a heart field with a stereoscopic microscope. After the lapse of 50, 55 and 60 hours from the start of incubation, 2 μl of the virus solution prepared in Example 2 was microinjected to the heart using a microinjector.

(Example 5) β-galactosidase activity measurement

After the lapse of 115 hours from the start of the incubation, the embryo was taken out from the eggshell, and washed with PBS (phosphate buffer solution) to remove a membrane enclosing the embryo. The removed embryo was finely sheared, and added with 0.8 ml of a reaction buffer (10 mM KCl, 1 mM $MgCl_2$, 0.1% Triton X-100 (product of Wako Pure Chemical Industries, Ltd.), 5 mM 2-mercaptoethanol (product of Wako Pure Chemical Industries, Ltd.), and 2mM phosphate buffer pH 7.5), and subjected to ultrasonic disruption to obtain a cell liquid.

0.6 ml of the cell liquid was incubated at 37°C for 10 minutes, and 0.1 ml of a liquid prepared by dissolving 4 mg/ml of o-nitrophenyl-β-D-galactopyranoside (ONPG) (product of Sigma Corporation) in 0.1M phosphate buffer (pH 7.5) warmed in advance was added. After the reaction, 0.3 ml of 1M $Na_2CO_3$ (product of Wako Pure Chemical Industries, Ltd.) was added and the wavelength strength at 420 nm was measured with an adsorption meter.

The β-galactosidase activity was expressed in terms of ONPG unit (1 unit: activity with which 1 μmol of o-nitrophenol is generated per minute). The experiment was carried out three times, and the average value thereof was used as the β-galactosidase activity.

The relation between the gene introduction time and β-galactosidase activity measurement result of chickens and quails was shown in Fig. 2 and Fig. 3. In quails, the β-galactosidase gene introduced after 48 hours of the incubation, and in chickens, that introduced after 55 hours were expressed stronger than those introduced before.

These findings suggest that the inactivation (silencing) mechanism of retrovirus for exogenous genes which the bird fertile eggs have is remarkable immediately after the fertilization, but becomes weakened with the time lapses. Thus, it becomes possible to produce a G0 transgenic chimera bird efficiently expressing the transgene without being inactivated by introducing an intended gene to a fertile egg after the lapse of a specific time, which depends on the bird species.

(Example 6) Efficiency of gene expression by virus titer

The 1 x $10^8$ cfu/ml virus solution prepared in Example 2 was diluted with dilution solvents (50 mM Tris-HCl (pH 7.8), 130 mM NaCl, and 1 mM EDTA solution) in three stages of 10-fold, 100-fold, and 1000-fold. Thus, 1 x $10^7$, 1 x $10^6$, and 1 x $10^5$ cfu/ml titer virus solutions were prepared. Quail fertile eggs were incubated, and to early development hearts after 48 hours, 2 pi of the prepared virus solutions were microinjected. In the same manner, as a control, 2 μl of the dilution solvent alone was injected to an early development heart after 48 hours.

After the lapse of 115 hours from the start of the incubation, the β-galactosidase activity was measured according to Example 5. The relation between the virus titer and gene expression result in quails was shown in Fig. 4.

In the same manner, chicken fertile eggs were incubated, and to early development hearts after 55 hours, 2 μl of the prepared virus solutions were microinjected. As a control, 2 μl of the dilution solvent alone was injected to an early development heart after 55 hours.

After the lapse of 115 hours from the start of the incubation, the β-galactosidase activity was measured according to Example 5. The relation between the virus titer and gene expression result in chickens was shown in Fig. 5.

In the quail and chicken injected with 1 x $10^8$ cfu/ml of virus, remarkable β-galactosidase activity was observed, and in those injected with the lower concentrations, the expression amounts were low. It was suggested that the virus titer greatly affects the expression of transgene, that is, for efficiently expressing the transgene with the G0 transgenic chimera bird of the invention, the use of high titer replication-defective vector is effective.

(Example 7) Transition ability of a human antibody to a quail and chicken egg

A mixture comprising human antibodies having three subclasses (IgG 1, 2, and 3) (product of Cosmo Bio Co., Ltd.) and the corresponding three species of antibody fragments (Fab-1, Fab-2, Fab-3, Fc-1, Fc-2, and Fc-3) (products of Cosmo Bio Co., Ltd.) were diluted with PBS so as to be 100 μg/ml. 100 μl of the diluted solutions were injected into veins under wings of quail adult birds (three birds) or chicken adult birds (three birds).

Eggs were collected from the next day to the 20th day of the antibody injection into veins, and the antibodies transferred into eggs were quantified. Egg yolk and albumen were diluted to 50% (W/V) and 10% (V/V) with PBS, respectively, and preserved in frozen state to be used as measurement samples.

(Example 8) Quantification of an antibody in eggs by ELISA method

An anti-human IgG antibody (product of Cosmo Bio Co., Ltd.) diluted with PBS was put into ELISA plates in 100 μg/well, and allowed to stand at 4°C overnight. Each plate was washed with 200 μl of PBS-0.05% Tween 20 solution three times, and then PBS-0.05% Tween 20 solution-2% skim milk was added in the wells in 150 μl/well.

After allowing the mixtures to stand at room temperature for 2 hours, the wells were washed with 200 μl of PBS-0.05% Tween 20 solution three times. Then, blood, albumen and egg yolk samples were placed therein in 120 μl and the mixtures were allowed to stand at 4°C overnight. After returning these ELISA plates to room temperature, each well was washed with PBS-Tween 20 solution three times, Peroxide (POD) labeled anti-human IgG antibody (product of Cosmo Bio Co., Ltd.) diluted with PBS-0.05% Tween 20 solution was put into each well in 100 μl/well, and allowed to stand for 1 hour at room temperature.

The wells were washed with PBS-0.05%Tween 20 solution four times, and 100 μl of a coloration liquid (prepared by dissolving 10 mg of o-phenylene diamine (product of Katayama Chemical Industry Co., Ltd.) in 1 ml of methanol, diluting with distilled water to be 100 ml, and adding 10 μl of hydrogen peroxide (product of Wako Pure Chemical Industries, Ltd.)) was added to the wells. Then, 50 μl of 8M sulfuric acid was added to quench the reaction, and the fluorescence intensity at 490nm was determined with a plate reader to calculate the concentration from the standard calibration curve. The result was obtained by averaging the antibody concentrations of the samples obtained from three quails and chickens.

The standard antibody for standard calibration-curve construction (product of Cosmo Bio Co., Ltd.) was diluted with 50% egg yolk-PBS (W/V).

The antibody concentrations accumulated in quail and chicken eggs were shown in Fig. 6 and 7. The Fab and Fc fragment concentrations accumulated in quail and chicken eggs were shown in Fig. 8, 9, 10 and 11.

In quails and chickens, it was found that human IgG antibody, while in subclass, human IgG2 and human IgG1, were efficiently accumulated in eggs. Moreover, since the Fc fragment showed high transition ability to eggs, it was suggested that Fc receptor intervenes the transition of human IgG.

(Example 9) Production of an anti-CD2 antibody expression vector construct

Vector constructs for anti-CD2 antibody expression pMSCV/GΔAH, pMSCV/GΔAL, and pMSCV/GΔALIH were produced as follows.

1. mRNA was obtained from human antibody (IgM)-producing hybridoma cell D253-15-6 (American Type Culture Collection HB-8789) using Quick Prep Micro mRNA Purification Kit (product of Pharmacia K.K.), and a cDNA library was prepared using First-Strand cDNA Synthesis Kit (product of Pharmacia K.K.) from the obtained mRNA. By PCR (94°C/l minute, 50°C/l minute, 72°C/1.5 minutes: 25 cycles; Taq DNA polymerase (product of PerkinElmer, Inc.)) using two chemosynthesis oligonucleotides 5'-atcctcgagaggccaaagtacagtg-3' (SEQ ID NO:7; underlined portion is XhoI restriction enzyme site) and 5'-cccggatccctaacactctcccctgttgaagct-3' (SEQ ID NO:8; underlined portion is BamHI restriction enzyme site) as primers, a gene fragment of human antibody L chain κ constant region (hCκ) was amplified from the above DNA library, cut with restriction enzymes XhoI and BamHI, and then inserted into XhoI and BamHI sites of plasmid pBluescript IIKS (-), (product of Stratagene) to produce plasmid pBlue/hCκ.

2. In the same manner, by PCR using two chemosynthesis oligonucleotides 5'-agcggccgctacaggtgtccactccgacatcgtgatgacccagtctcc-3' (SEQ ID NO:9; underlined portion is NotI restriction enzyme site) and 5'-cctctcgaggatagaagttattcagcaggcacac-3[1] (SEQ ID NO:10; underlined portion is XhoI restriction enzyme site) as primers, a gene fragment of human antibody L chain variable region (hVL) was amplified from the above cDNA library, cut with restriction enzymes NotI and XhoI, and then inserted into NotI and XhoI sites of pBluescript IIKS (-) to produce plasmid pBlue/hVL.

3. In the same manner, by PCR using two chemosynthesis oligonucleotides 5'-acctcgagcgtggccgttggctgcctcgcaca-3' (SEQ ID NO:11; underlined portion is XhoI restriction enzyme site) and 5'-actaagcttacgttgtacagggtgggtttacc-3' (SEQ ID NO:12; underlined portion is HindIII restriction enzyme site) as primers, a gene fragment of human antibody H chain μ constant region (hCμ) was amplified from the above cDNA library, cut with restriction enzymes XhoI and HindIII, and then inserted into XhoI and HindIII sites of pBluescript IIKS (-) to produce plasmid pBlue/hCμ.

4. In the same manner, by PCR using two chemosynthesis oligonucleotides 5'-agcggccgctacaggtgtccactccgaggtgcagctggtggagtctgg-3' (SEQ ID NO:13; underlined portion is NotI restriction enzyme site) and 5'-cacgctcgaggtatccgacggggaattctcacagga-3' (SEQ ID NO:14; underlined portion is XhoI restriction enzyme site) as primers, a gene fragment of human antibody H chain variable region (hVH) was amplified from the above cDNA library, cut with restriction enzymes NotI and XhoI, and then inserted into NotI and XhoI sites of pBluescript IIKS (-) to produce plasmid pBlue/hVH.

5. From pBlue/hCκ, hCκ gene fragment was cut using restriction enzymes XhoI and BamHI, and then inserted into XhoI and BamHI sites of plasmid pCEP4 (product of Invitrogen Corporation) to produce plasmid pCEP4/hCκ.

6. Two chemosynthesis oligonucleotides 5'-cccaagcttgatctccactgggatggtgggggccctcctcttgctgctg-3' (SEQ ID NO:15; underlined portion is HindIII restriction enzyme site) and 5'-cccggatcctcagtcaaggcgccttcgcatgaagaggccgatccccagggccaccacca gcagcaagaggagggcccc-3' (SEQ ID NO:16; underlined portion is BamHI restriction enzyme site) were annealed over 21 bps with a complementary 3' end to produce a gene fragment of epidermal growth factor receptor membrane penetration region (TM) by DNA double strand synthesis reaction using T4 DNA polymerase (Takara Shuzo Co., Ltd.). The obtained TM gene fragment was treated with restriction enzymes HindIII and BamHI, and then inserted into HindIII and BamHI sites of pBluescript IIKS (-) to produce plasmid pBlue/TM.

7. From pBlue/hCμ, hCμ gene fragment was cut using restriction enzymes XhoI and HindIII, and then inserted into XhoI and HindIII sites of pBlue/TM to produce plasmid pBlue/hCμTM.

8. From pBlue/hCμTM, a fragment containing a series of hCμ genes and TM gene was cut using restriction enzymes XhoI and BamHI, and then inserted into XhoI and BamHI sites of pCEP4 to produce plasmid pCEP4/hCμTM.

9. pCEP4/hCμTM was cut with restriction enzyme BamHI, and the end was treated to be smooth with T4 DNA polymerase to produce plasmid pCEP4/hCμTMΔB by self ligation.

10. By site-specific mutagenesis using a chemosynthesis oligonucleotide 5'-tgaagacagatggcgccgccacagttcgttt-3' (SEQ ID NO:17; underlined portion is NarI restriction enzyme site), restriction enzyme NarI site was introduced into 3' end of hVL contained in pBlue/hVL without changing the amino acid codons to produce plasmid pBlue/hVLN.

11. By site-specific mutagenesis using a chemosynthesis oligonucleotide 5'-tggggcggatgcggatcctgaggagacggt-3' (SEQ ID NO:18; underlined portion is BamHI restriction enzyme site), restriction enzyme BamHI site was introduced into 3' end of hVL contained in pBlue/hVL without changing the amino acid codons to produce plasmid pBlue/hVHB.

12. mRNA was obtained from anti-human CD2 mouse antibody-producing hybridoma cell TS2/18.1.1 (American Type Culture Collection HB-195) using Quick Prep Micro mRNA Purification Kit, and a cDNA library was prepared using First-Strand cDNA Synthesis Kit from the obtained mRNA. By PCR using two chemosynthesis oligonucleotides 5'-cgcggccgcctcagggaaagtttgaagatg-3' (SEQ ID NO:19; underlined portion is NotI restriction enzyme site) and 5'-cggcgccgccacagtccgttttatttccagcttggt-3' (SEQ ID NO:20; underlined portion is NarI restriction enzyme site) as primers, a gene fragment of mouse antibody L chain variable region (mVL) was amplified from the above cDNA library, cut with restriction enzymes NotI and NarI, and then inserted into NotI and NarI sites of pBlue/hVLN to produce plasmid pBlue/mVL.

13. In the same manner, by PCR using two chemosynthesis oligonucleotides 5'-cgcggccgcgaacacggamccctcaccatg-3' (SEQ ID NO:21; underlined portion is NotI restriction enzyme site) and 5'-cggatcctgcagagacagtgaccagagt-3' (SEQ ID NO:2.2; underlined portion is BamHI restriction enzyme site) as primers, a genre fragment of mouse antibody H chain variable region (mVH) was amplified from the above cDNA library, cut with restriction enzymes NotI and BamHI, and then inserted into NotI and BamHI sites of pBluescript IIKS (-) to produce plasmid pBlue/mVH.

14. From pBlue/mVL, mVL gene fragment was cut with restriction enzymes NotI and XhoI, and then inserted into NotI and XhoI sites of pCEP4/hCκ to produce plasmid pCEP4/IgLκ.

15. From pBlue/hVHB, hVH gene fragment was cut with restriction enzymes NotI and XhoI, and then inserted into NotI and XhoI sites of pCEP4/hCμTMΔB to produce plasmid pCEP4/hIgHμTM.

16. From pBlue/mVH, mVH gene fragment was cut with restriction enzymes NotI and BamHI, and then linked to a vector fragment of pCEP4/hIgHμTM treated with restriction enzymes NotI and BamHI to produce plasmid pCEP4/IgHμTM.

17. From plasmid pMSCVneo (product of BD Biosciences Clontech), a fragment containing a series of murine phosphoglycerate kinase (PGK) promoters and $Neo^r$ gene was removed with restriction enzymes BglII and BamHI, and plasmid pMSCV was produced by self ligation of the remained vector fragment.

18. From plasmid pGREEN LANTERN-1 (product of Gibco BRL), GFP gene fragment was cut with restriction enzyme NotI, and then inserted into NotI site of pZeoSV2 (+). The plasmid having the structure of GFP gene being inserted in the same direction as T7 promoter was named pZeo/GFP.

19. From pZeo/GFP, GFP gene fragment was cut with restriction enzymes EcoRI and XhoI, and then linked to a vector fragment of pMSCV treated with restriction enzymes EcoRI and XhoI to produce plasmid pMSCV/G.

20. mRNA was obtained from human antibody (IgG1) producing myeloma cell IM-9 (Japanese Collection of Research Bioresources 0024) using mRNA isolation kit (product of Roche Ltd.), and a cDNA library was prepared using ReverTra Ace (product of Toyobo Co., Ltd.) from the obtained mRNA. By PCR (95°C/2 minutes, 52°C/30 seconds, 74°C/3 minutes: 30 cycles; Pfu DNA polymerase (product of Promega Corporation))using two chemosynthesis oligonucleotides 5'-caagcttcaagggcccat-3' (SEQ ID NO:23) and 5'-atttacccggagacaggga-3' (SEQ ID NO:24), a gene fragment of human antibody H chain γ1 constant region (hCγ1) was amplified from the above cDNA library. Furthermore, by PCR (94°C/15 seconds, 58°C/30 seconds, 68°C/l minute: 30 cycles; KOD-plus-DNA polymerase) using two chemosynthesis oligonucleotides 5'-ataggatccgctagcttcaagggcccatcg-3' (SEQ ID NO:25; underlined portion is BamHI restriction enzyme site) and 5'-agcaagctttcatttacccggagacaggga-3' (SEQ ID NO:26; underlined portion is HindIII restriction enzyme site), as primers, hCγ1 gene fragment was amplified from the above PCR product, cut with restriction enzymes BamHI and HindIII, and then inserted into BamHI and HindIII sites of pBluescript IISK (+) to produce plasmid pBlue/hCγ1.

21. From pCEP4/IgHμTM, mVH gene fragment was cut with restriction enzymes HindIII and BamHI. From pBlue/hCγ1, hCγ1 gene fragment was cut with restriction enzymes BamHI and HindIII. To a vector fragment of plasmid pETBlue-2 (product of Novagen, Inc.) treated with restriction enzyme HindIII, the above two cut fragments were linked to produce plasmid pETBlue/IgHγ1.

22. From pETBlue/IgHγ1, a gene fragment of antibody H chain γ1 (IgHγ1) was cut with restriction enzyme HindIII, and inserted into HindIII site of pMSCV/G. The plasmid having the structure of IgHγ1 gene being inserted in the same direction as GFP gene was named pMSCV/GH.

23. By PCR (94°C/15 seconds, 50°C/30 seconds, 68°C/l minute: 10 cycles; 94°C/15 seconds, 62°C/30 seconds, 68°C/l minute: 30 cycles) using two chemosynthesis oligonucleotides 5'-acgcgtcgacgtgcatgcacgctcattg-3' (SEQ ID NO:27; underlined portion is SalI restriction enzyme site) and 5'-acgcgtcgacaacgcagcgactcccg-3' (SEQ ID NO:28; underlined portion is SalI restriction enzyme site) as primers, ΔAct promoter fragment was amplified from pMiwZ, cut with restriction enzyme SalI, and inserted into SalI site of pETBlue-2 to produce plasmid pETBlue/ΔAct.

24. From pETBlue/Δact, ΔAct promoter fragment was cut with restriction enzyme SalI, and inserted into XhoI site of pMSCV/GH. The plasmid having the structure of ΔAct promoter being inserted in the same direction as IgHγ1 gene was named pMSCV/GΔAH.

25. By PCR (95°C/30 seconds, 50°C/30 seconds, 794°C/2 minutes: 10 cycles; 95°C/30 seconds, 60°C/30 seconds, 74°C/2 minutes: 30 cycles; Pfu DNA polymerase) using two chemosynthesis oligonucleotides 5'-aatgtcgacatggtgtccacttctcagctc-3' (SEQ ID NO:29; underlined portion is SalI restriction enzyme site) and 5'-ttcgtcgacctaacactctcccctgttgaa-3' (SEQ ID NO:30; underlined portion is SalI restriction enzyme site) as primers, a gene fragment of antibody L chain κ (IgLκ) was amplified from pCEP4/IgLκ, cut with restriction enzyme SalI, and then inserted into SalI site of pETBlue-2 to produce plasmid pETBlue/IgLκ.

26. From pETBlue/Δ Act, ΔAct promoter fragment was cut with restriction enzyme SalI, and then inserted into XhoI site of pMSCV/G. The plasmid having the structure of ΔAct promoter being inserted in the same direction as GFP gene was named pMSCV/GΔA.

27. From pETBlue/IgLκ, IgLκ gene fragment was cut with restriction enzyme SalI, and then inserted into SalI site of pMSCV/GΔA. The plasmid having the structure of IgLκ gene fragment being inserted in the same direction as ΔAct promoter was named pMSCV/GΔAL.

28. By PCR (94°C/15 seconds, 60°C/30 seconds, 68°C/l minute: 30 cycles) using two chemosynthesis oligonucleotides 5'-acgcgtcgaccgcccctctccctccccc-3' (SEQ ID NO:31; underlined portion is SalI restriction enzyme site) and 5'-ccgctcgagattatcatcgtgtttttcaaaggaaaaccacgtc-3' (SEQ ID NO:32; underlined portion is XhoI restriction enzyme site) as primers, IRES fragment was amplified from plasmid pLXIN (product of BD Biosciences Clontech), cut with restriction enzymes SalI and XhoI, and then inserted into SalI and XhoI sites of pETBlue-2 to produce plasmid pETBlue/IRES.

29. From pETBlue/IRES, IRES fragment was cut with restriction enzymes SalI and XhoI, and then inserted into SalI site of pMSCV/GΔAH. The plasmid having the structure of IRES being inserted in the same direction as IgHγ1 gene was named pMSCV/GΔAIH.

30. From pETBlue/IgLκ, IgLκ gene fragment was cut with restriction enzyme SalI, and then inserted into SalI site of pMSCV/GΔAIH. The plasmid having the structure of IgLκ gene fragment being inserted in the same direction as ΔAct promoter was named pMSCV/GΔALIH.

The thus produced vector constructs of replication-defective retrovirus vector pMSCV/GΔAH, pMSCV/GΔAL and pMSCV/GΔALIH were shown in Fig. 12.

(Example 10) Production of anti-CD2 antibody expressing G0 transgenic chimera quail

According to Example 2, three species of retrovirus vectors were prepared from vector constructs pMSCV/GΔAH, pMSCV/GΔAL and pMSCV/GΔALIH. The titers of these retrovirus vectors were measured and found to be $10^8$ cfu/ml to $10^9$ cfu/ml.

The obtained retrovirus vectors were microinjected to hearts of quail fertile eggs after 36 hours from the start of incubation according to Example 3, and the eggs were incubated while rotating the eggs at 90 degrees every 15 minutes at 37.9°C and humidity of 65%.

In order to cause an antibody comprising a light chain and heavy chain to be expressed in transgenic animals, presumable are a method comprising introducing a vector expressing a light chain and vector expressing a heavy chain individually, and a method comprising dividing genes expressing a light chain and genes expressing a heavy chain with a sequence such as IRES, and introducing thereof as the same vector. Thus, injection was carried out separately in a case where pMSCV/GΔAH and pMSCV/GΔAL were infected at the same time (Example 11 and Experiment Example 1), and a case where a vector prepared by pMSCV/GΔALIH was introduced alone (Example 11 and Experiment Example 2).

After 48 hours from the start of incubation, that the generation normally proceeded was confirmed, and then the virus-introduced embryo was transferred to an S size hen egg in which a 4 cm-diameter circle was holed on the blunt-round end. With placing the embryo upside to be exposed to air, 0.5 ml of a calcium lactate (product of Sigma Corporation) solution suspended in albumen at the concentration of 50 mg/ml was added, and the egg was sealed with wrap using albumen as a paste. The egg was again placed in an incubator, and incubated for 13 days at 37.9°C and humidity of 65% with rotations at 60 degrees every hour. Then, the rotation was stopped and the egg was allowed to stand. When the embryo shifted to pulmonary respiration, a small hole was made on wrap with a needle to assist respiration. When blood in chorioallantois reduced, a baby chick was taken out from the incubator and hatched.

(Example 11) Determination of the concentration of anti-CD2 antibody in serum and egg

G0 transgenic chimera quails hatched in Example 10 were bred for one month to grow baby chicks. After 30 and 60 days, blood was sampled from veins under wings of the grown G0 transgenic quails to obtain blood samples. The obtained blood was centrifuged for 10 minutes at 15,000 rpm, and the amount of anti-CD2 antibody was determined from serum obtained as supernatant.
After 1.5 months from the hatch, eggs were collected from female transgenic quails which started egg laying, and the amount of anti-CD2 antibody in albumen and egg yolk prepared according to Example 7 was determined by ELISA method according to Example 8.
The quantification results of Experiment Example 1 and Experiment Example 2 were shown.

(Experiment Example 1)

The G0 transgenic chimera quail (individual identification number #1113) simultaneously infected with vectors (3 to 4 x $10^8$ cfu/ml) prepared from pMSCV/GΔAH and pMSCV/GΔAL expressed anti-CD2 antibody at concentrations of 0.6 μg/ml in egg yolk, and 0.5 μg/ml in albumen.

(Experiment Example 2)

The G0 transgenic chimera quail (#4202) introduced a vector prepared from pMSCV/GΔALIH (5 x $10^8$ cfu/ml) alone expressed 5.2 μg/ml of anti-CD2 antibody in serum.

(Example 12) Production of an scFv-Fc antibody expression vector construct

ScFv-Fc antibody expression vector construct pMSCV/scFv-Fc was produced as follows.

1. Two chemosynthesis oligonucleotides having phosphorylated 5' ends 5'-ctagaccatgaggtctttgctaatcttggtgctttgcttcctgcccctggctgctctgg gg-3' (SEQ ID NO:33; ctaga is XbaI recognition site end, and gg is HaeIII recognition site end) and 5'-ccccagagcagccaggggcaggaagcaaagcaccaagattagcaaagacctcatggt-3' (SEQ ID NO:34; cc is XbaI recognition site end, and t is HaeIII recognition site end) were annealed to prepare a gene fragment of lysozyme secretion signal. By PCR (94°C/15 seconds, 58°C/30 seconds, 68°C/l minute: 30 cycles; KOD-Plus-DNA polymerase) using two chemosynthesis oligonucleotides 5'-gcgtttaaagtgacgttggacgtccg-3' (SEQ ID NO:35; tttaaa is DraI restriction enzyme site) and 5'-attaggatccgcgcttaaggacggtcagg-3' (SEQ ID NO:36; ggatcc is BamHI restriction enzyme site) as primers, an scFv gene fragment was amplified from plasmid pPDS/scFv containing a gene of a single strand antibody (scFv) prepared from HUC2-13 cell chicken antibody variable region gene (Nakamura et al., 2000, and Cytotechnology 32: 191-198), and cut with restriction enzymes DraI and BamHI. The above two prepared fragments were inserted into XbaI and BamHI sites of pBluescript IISK (+) to produce plasmid pBlue/scFv.

2. The scFv gene fragment was cut with restriction enzymes NotI and BamHI from pBlue/scFv, and then inserted into NotI and BamHI sites of pCEP4 to produce plasmid pCEP4/scFv.

3. mRNA was obtained from human IgG1-producing myeloma cell IM-9 using the mRNA isolation kit, and a cDNA library was prepared using ReverTra Ace from the obtained mRNA. By PCR (95°C/2 minutes, 52°C/30 seconds, 74°C/3 minutes: 30 cycles; Pfu DNA polymerase) using two chemosynthesis oligonucleotides 5'-caagcttcaagggcccat-3' (SEQ ID NO:23) and 5'-atttacccggagacaggga-3' (SEQ ID NO:24) as primers, hCγ1 gene fragment was amplified from the above cDNA library. Furthermore, by PCR (94°C/15 seconds, 58°C/30 seconds, 68°C/l minute: 30 cycles; KOD-plus-DNA polymerase) using two chemosynthesis oligonucleotides 5'-attaggatccgagcccaaatcttgtgacaaaactc-3' (SEQ ID NO:37; ggatcc is BamHI restriction enzyme site) and 5'-agcaagctttcatttacccggagacaggga-3' (SEQ ID NO:26; aagctt is HindIII restriction enzyme site) as primers, a gene fragment of human antibody H chain γ1 Fc region (Fc) was amplified from the above PCR product, cut with restriction enzymes BamHI and HindIII, and then inserted into BamHI and HindIII sites of pBluescript IISK (+) to produce plasmid pBlue/Fc.

4. From pCEP4/scFv, an scFv gene fragment was cut with restriction enzymes HindIII and BamHI. From pBlue/Fc, Fc gene fragment was cut with restriction enzymes BamHI and HindIII. The above two cut fragments were inserted into HindIII site of pBluescript IISK (+) to produce plasmid pBlue/scFv-Fc.

5. From pBlue/scFv-Fc, a gene fragment having the structure of human antibody H chain γ1·Fc being linked to a chicken single chain antibody variable region (scFv-Fc) was cut with restriction enzyme HindIII, and linked to a vector fragment of pMSCV/GΔAH treated with restriction enzyme HindIII. The plasmid having the structure of an scFv-Fc gene being linked in the same direction as ΔAct promoter was named pMSCV/GΔAscFv-Fc.

The structure of thus produced vector construct pMSCV/GΔAscFv-Fc of the replication-defective retrospective virus vector was shown in Fig. 13.

(Example 13) Production of scFv-Fc antibody expressing G0 transgenic chimera quails

According to Example 2, a retrovirus vector was prepared from vector construct pMSCV/GΔAscFv-Fc. The titer of this retrovirus vector was determined and found to be $10^8$ cfu/ml to $10^9$ cfu/ml.

According to Example 3, the obtained virus vector solution was microinjected into hearts of quail fertile eggs after 36 hours from the start of incubation. The eggs were hatched according to Example 10 to produce G0 transgenic chimera quails.

(Example 14) Determination of the concentration of scFv-Fc in serum and egg

G0 transgenic chimera quails produced in Example 13 were bred for one month to grow baby chicks. After the lapse of 30 and 60 days, blood was sampled from veins under wings of the grown G0 transgenic chimera quails (individual identification number #3303, #3306, #3310, #3311, and #3313) to obtain blood samples. The obtained blood was centrifuged at 15,000rpm for 10 minutes, and the amount of scFv-Fc antibodies was determined from serum obtained as supernatant.

Eggs were collected from female transgenic quails which started egg laying (individual identification number #3310) after 1.5 months from the hatch, and the amount of scFv-Fc antibody in albumen and egg yolk prepared according to Example 7 was quantitated by ELISA method according to Example 8.

The standard calibration curve was constructed using a purified scFv-Fc. Vector construct pMSCV/scFv-Fc produced in Example 12 was introduced into GP293 cell by the lipofection method, and the culture supernatant was centrifuged at 3000 rpm and 4°C for 10 minutes to remove solid matters. While cooling, this supernatant was stirred and gradually added with finely crushed ammonium sulfate so as to be 50% saturation (313 g ammonium sulfate/1000 ml water) to precipitate protein. The mixture was allowed to stand at 4°C overnight, and centrifuged at 15,000 rpm and 4°C for 10 minutes to completely precipitate the protein. The precipitated protein was then dissolved in a small amount of PBS, and dialyzed by 2 L of PBS three times to remove ammonium sulfate.

The initial washing of the protein G column for purification (product of Perseptive Biosystems, Inc.) was carried out using 10 mL of a binding buffer ($NaHPO_4 \cdot 2H_2O$ 1.56 g/l, $NaHPO_4 \cdot 12H_2O$ 7.16 g/l), 10 ml of a wash buffer (acetic acid 20%, distilled water 80%), and 10 ml of the binding buffer (flow rate 2 ml/min.) in this order. The protein liquid dissolved in PBS was flowed at 1 ml/min. and an scFv-Fc was adsorbed on a column. By flowing 20 ml of the binding buffer at 1.7 ml/min., unnecessary protein was removed, and an elution buffer (prepared with glycine 7.507 g/l and 2N HCl to pH of 2.5 to 3.0) at 1.5 ml/min. to elute the scFv-Fc.

The eluted fraction was dialyzed with PBS (2L) three times to obtain a purified scFv-Fc, and the protein concentration was quantitated from the adsorption at a wavelength of 280 nm.

The amount of the scFv-Fc in blood serum sampled after 30 and 60 days of the G0 transgenic chimera quails produced in Example 14 was shown in Fig. 14. The G0 transgenic chimera quail introduced an scFv-Fc antibody expression gene expressed about 2 mg/ml to 4 mg/ml of antibody in serum at 30th day, and three of five birds also showed the same extent of expression amount.

From the day that the G0 transgenic chimera quail (#3310) started egg laying, the amount of scFv-Fc in egg yolk and albumen was shown in Fig. 15. The antibody was expressed in about 500 μg/ml to 1 mg/ml in albumen and egg yolk. Even there was a slight variation from the start of egg laying to the 17th day, a stable expression amount was maintained.

(Example 15) Confirmation of the scFv-Fc structure

From 1 ml of G0 transgenic chimera quail serum produced in Example 13, an scFv-Fc was purified from ammonium sulfate precipitate and the protein G column according to Example 10. The purified scFv-Fc was analyzed by SDS-PAGE, and the result was shown in Fig. 16. From the untreated lane, the scFv-Fc molecular weight of about 120 kDa was shown. Since the molecular weight of the scFv-Fc subjected to reduction treatment was about a half of the untreated ones (about 60 kDa), it was found that the scFv-Fc produced by a G0 transgenic chimera quail forms a dimer by an S-S bond. These corresponded to the structural feature of the scFv-Fc having a cysteine residue involved with an S-S bond in Fc portion, thus it was suggested that scFv-Fc produced by a G0 transgenic chimera quail retained the correct structure.

(Example 16) Production of TNFR-Fc fusion protein expressing G0 transgenic chimera chickens

According to Example 9, TNFR-Fc expression vector construct was produced, and a retrovirus vector was produced according to Example 2. The titer of this retrovirus vector was 1.7 x $10^7$ cfu/ml.

The obtained virus vector solution was microinjected to chicken fertile egg hearts after 55 hours from the start of incubation according to Example 4, and hatched according to Example 10 to produce G0 transgenic chimera chickens.

The solution was injected to eight fertile eggs, and four birds were hatched. TNFR-Fc in serum was quantitated according to Example 14, and TNFR-Fc of 50 μg/ml at the maximum was found to be expressed.

INDUSTRIAL APPLICABILITY

The G0 transgenic chimera bird of the present invention can efficiently express a gene introduced using a replication-defective retrovirus vector without causing inactivation. Moreover, the production method of a G0 transgenic chimera bird of the present invention makes it possible to introduce a gene of a chimera antibody, for example of an scFv-Fc antibody, and to produce birds capable of efficiently expressing the antibody in blood and eggs. Furthermore, the production method of an antibody of the present invention comprises producing a G0 transgenic chimera bird producing a chimera antibody, for example an scFv-Fc antibody, recovering and purifying the antibody from serum and eggs of the bird, thus the efficient production of an antibody becomes possible.

## Claims

1. A G0 transgenic chimera bird which is introduced an exogenous antibody gene with a replication-defective retrovirus vector, and produces an antibody derived from the transgene in at least one of blood, albumen, and egg yolk, wherein the content of said antibody is not less than 20 μg/ml in blood, or not less than 5 μg/ml in albumen.

2. The G0 transgenic chimera bird according to Claim 1, wherein a class of a constant region of the antibody is human IgG.

3. The G0 transgenic chimera bird according to Claim 1, wherein a subclass of a constant region of the antibody is human IgG1.

4. The G0 transgenic chimera bird according to Claim 1, wherein the constant region of the antibody is quail IgG, chicken IgG, or mouse IgG.

5. The G0 transgenic chimera bird according to any one of Claims 1 to 4, wherein the antibody gene is controlled by a constitutive promoter.

6. The G0 transgenic chimera bird according to Claim 5, wherein the constitutive promoter is chicken beta-actin promoter.

7. The G0 transgenic chimera bird according to any one of Claims 1 to 6, wherein the retrovirus vector is a vector derived from Moloney murine leukemia virus.

8. The G0 transgenic chimera bird according to any one of Claims 1 to 7, wherein the retrovirus vector is a VSV-G pseudo type one.

9. The G0 transgenic chimera bird according to any one of Claims 1 to 8, wherein the bird is a chicken or quail.

10. The G0 transgenic chimera bird according to any one of Claims 1 to 9, wherein the antibody is a chimera antibody.

11. The G0 transgenic chimera bird according to Claim 10, which contains not less than 0.1 μg/ml of the antibody in egg yolk.

12. The G0 transgenic chimera bird according to Claim 11, which contains not less than 1 μg/ml of the antibody in egg yolk.

13. The G0 transgenic chimera bird according to any one of Claims 1 to 9, wherein the antibody is a scFv-Fc antibody.

14. The G0 transgenic chimera bird according to Claim 13, which contains not less than 20 μg/ml of the antibody in blood.

15. The G0 transgenic chimera bird according to Claim 14, which contains not less than 2000 μg/ml of the antibody in blood.

16. The G0 transgenic chimera bird according to Claim 13, which contains not less than 5 μg/ml of the antibody in albumen.

17. The G0 transgenic chimera bird according to Claim 16, which contains not less than 500 μg/ml of the antibody in albumen.

18. The G0 transgenic chimera bird according to Claim 13, which contains not less than 5 μg/ml of the antibody in egg yolk.

19. The G0 transgenic chimera bird according to Claim 18, which contains not less than 500 μg/ml of the antibody in egg yolk.

20. A production method of a G0 transgenic chimera bird which comprises incubating a bird fertile egg, infecting an early embryo after the lapse of 24 hours or more from the start of the incubation with a replication-defective retrovirus vector, and then hatching the embryo,
wherein a gene sequence coding for an antibody gene is contained in a transgene incorporated into a replication-defective retrovirus vector.

21. The production method of a G0 transgenic chimera bird according to Claim 20, which comprises incubating a bird fertile egg, microinjected with a replication-defective retrovirus vector to a heart or blood vessel formed in the early embryo.

22. The production method of a G0 transgenic chimera bird according to Claim 20, which comprises incubating a bird fertile egg, microinjected with a replication-defective retrovirus vector to a heart or blood vessel formed in the early embryo formed after the lapse of 24 hours or more from the start of the incubation.

**23.** The production method of a G0 transgenic chimera bird according to any one of Claims 20 to 22, microinjected with a replication-defective retrovirus vector having the titer of not less than 1 x $10^7$ cfu/ml.

**24.** The production method of a G0 transgenic chimera bird according to Claim 23, microinjected with a replication-defective retrovirus vector having the titer of not less than 1 x $10^8$ cfu/ml.

**25.** The production method of a G0 transgenic chimera bird according to Claim 24, microinjected with a replication-defective retrovirus vector having the titer of not less than 1 x $10^9$ cfu/ml.

**26.** The production method of a G0 transgenic chimera bird according to any one of Claims 20 to 25, wherein the retrovirus vector is a vector derived from Moloney murine leukemia virus.

**27.** The production method of a G0 transgenic chimera bird according to any one of Claims 20 to 26, wherein the retrovirus vector is a VSV-G pseudo type one.

**28.** The production method of a G0 transgenic chimera bird according to any one of Claims 20 to 27, wherein the bird is a chicken or quail.

**29.** The production method of a G0 transgenic chimera bird according to Claims 20 to 28, wherein the gene sequence coding for an antibody gene is controlled by chicken beta-actin promoter.

**30.** The production method of a G0 transgenic chimera bird according to Claims 20 to 29, wherein the antibody gene is a chimera antibody gene.

**31.** The production method of a G0 transgenic chimera bird according to Claims 20 to 30, wherein the antibody gene is an scFv-Fc antibody gene.

**32.** A production method of an antibody which comprises producing the G0 transgenic chimera bird by the production method according to any one of claims 20 to 31, and recovering the antibody from blood and/or an egg of said G0 transgenic chimera bird.

**33.** A G0 transgenic chimera bird which is produced by the method according to any one of Claims 20 to 31, wherein the content of a protein derived from the transgene is not less than 20 μg/ml in blood, or not less than 5 μg/ml in albumen.

**34.** A production method of a transgenic bird, which comprises mating the G0 transgenic chimera bird according to Claim 33 with a mating type allogeneic bird, and then hatching the egg.

**35.** The production method of a transgenic bird according to Claim 34, wherein the mating type allogeneic bird is the G0 transgenic bird according to Claim 33 or an offspring thereof.

**36.** A production method of a transgenic bird, which comprises further mating the G1 transgenic bird which is obtained by the method according to Claim 34, and then hatching the egg.

**37.** A transgenic bird or an offspring thereof, which is obtainable by the method according to claims 34 to 36, wherein the content of the antibody is not less than 20 μg/ml in blood, or not less than 5 μg/ml in albumen.

**38.** The transgenic bird according to Claim 37,
which is a G1 transgenic bird,
wherein the mating type allogeneic bird is the G0 transgenic bird according to Claim 33 or an offspring thereof.

**39.** A production method of a protein, which comprises extracting the objective protein from a somatic cell, blood or an egg of the transgenic bird produced by the method according to Claims 34 to 36.

**40.** An egg laid by the transgenic bird according to Claims 37 or 38, which contains not less than 1 mg of a heterogeneous protein derived from a transgene.

**41.** The egg according to claim 40, which contains not less than 20 mg of a heterogeneous protein derived from a transgene.

**42.** The egg according to claim 41, which contains not less than 100 mg of a heterogeneous protein derived from a transgene.

**43.** The egg according to claim 42, which contains not less than 200 mg of a heterogeneous protein derived from a transgene.

## Patentansprüche

**1.** G0-transgener chimärer Vogel, welchem mit einem replikationsdefekten Retrovirusvektor ein exogenes Antikörpergen hinzugefügt wurde und welcher einen von dem Transgen stammenden Antikörper in zumindest einem von Blut, Albumen und Eigelb herstellt, wobei die Menge des Antikörpers nicht weniger als 20 μg/ml im Blut oder nicht weniger als 5 μg/ml im Albumen ist.

**2.** G0-transgener chimärer Vogel gemäß Anspruch 1, wobei eine Klasse einer konstanten Region des Antikörpers humanes IgG ist.

**3.** G0-transgener chimärer Vogel gemäß Anspruch 1, wobei eine Subklasse einer konstanten Region des Antikörpers humanes IgG1 ist.

**4.** G0-transgener chimärer Vogel gemäß Anspruch 1, wobei die konstante Region des Antikörpers Wachtel-IgG, Hühner-IgG oder Mäuse-IgG ist.

**5.** G0-transgener chimärer Vogel gemäß einem der Ansprüche 1 bis 4, wobei das Antikörpergen durch einen konstitutiven Promotor kontrolliert wird.

**6.** G0-transgener chimärer Vogel gemäß Anspruch 5, wobei der konstitutive Promotor ein Hühner-beta-Aktin-Promotor ist.

**7.** G0-transgener chimärer Vogel gemäß einem der Ansprüche 1 bis 6, wobei der Retrovirusvektor ein Vektor ist, welcher vom Moloney-Maus-Leukämie-Virus stammt.

**8.** G0-transgener chimärer Vogel gemäß einem der Ansprüche 1 bis 7, wobei der Retrovirusvektor ein VSV-G-Pseudotyp ist.

**9.** G0-transgener chimärer Vogel gemäß einem der Ansprüche 1 bis 8, wobei der Vogel ein Huhn oder eine Wachtel ist.

**10.** G0-transgener chimärer Vogel gemäß einem der Ansprüche 1 bis 9, wobei der Antikörper ein chimärer Antikörper ist.

**11.** G0-transgener chimärer Vogel gemäß Anspruch 10, welcher nicht weniger als 0,1 μg/ml des Antikörpers im Eigelb enthält.

**12.** G0-transgener chimärer Vogel gemäß Anspruch 11, welcher nicht weniger als 1 μg/ml des Antikörpers im Eigelb enthält.

**13.** G0-transgener chimärer Vogel gemäß einem der Ansprüche 1 bis 9, wobei der Antikörper ein scFv-Fc-Antikörper ist.

**14.** G0-transgener chimärer Vogel gemäß Anspruch 13, welcher nicht weniger als 20 μg/ml des Antikörpers im Blut enthält.

**15.** G0-transgener chimärer Vogel gemäß Anspruch 14, welcher nicht weniger als 2.000 μg/ml des Antikörpers im Blut enthält.

**16.** G0-transgener chimärer Vogel gemäß Anspruch 13, welcher nicht weniger als 5 μg/ml des Antikörpers im Albumen enthält.

**17.** G0-transgener chimärer Vogel gemäß Anspruch 16, welcher nicht weniger als 500 μg/ml des Antikörpers im Albumen enthält.

**18.** G0-transgener chimärer Vogel gemäß Anspruch 13, welcher nicht weniger als 5 μg/ml des Antikörpers im Eigelb enthält.

**19.** G0-transgener chimärer Vogel gemäß Anspruch 18, welcher nicht weniger als 500 μg/ml des Antikörpers im Eigelb enthält.

**20.** Herstellungsverfahren eines G0-transgenen chimären Vogels, umfassend das Inkubieren eines fertilen Vogeleis, das Infizieren eines frühen Embryos nach Ablauf von 24 Stunden oder mehr seit dem Beginn der Inkubation mit einem replikationsdefekten Retrovirusvektor und anschließend das Ausschlüpfen des Embryos,
wobei die Gensequenz, welche für ein Antikörpergen kodiert in einem Transgen enthalten ist, welches in einem replikationsdefekten Retrovirusvektor eingefügt wurde.

**21.** Herstellungsverfahren eines G0-transgenen chimären Vogels gemäß Anspruch 20, welches das Inkubieren eines fertilen Vogeleis umfasst, in welches ein replikationsdefekter Retrovirusvektor in ein Herz oder Blutgefäß, welches sich im frühen Embryo gebildet hat, mikroinjiziert wurde.

**22.** Herstellungsverfahren eines G0-transgenen chimären Vogels gemäß Anspruch 20, welches das Inkubieren eines fertilen Vogeleis umfasst, in welches ein replikationsdefekter Retrovirusvektor in ein Herz oder Blutgefäß, welches sich im frühen Embryo gebildet hat, nach dem Ablauf von 24 Stunden oder mehr seit dem Beginn der Inkubation, mikroinjiziert wurde.

**23.** Herstellungsverfahren eines G0-transgenen chimären Vogels gemäß einem der Ansprüche 20 bis 22, mikroinjiziert mit einem replikationsdefekten Retrovirusvektor mit einem Titer von nicht weniger als $1 \times 10^7$ cfu/ml.

**24.** Herstellungsverfahren eines G0-transgenen chimären Vogels gemäß Anspruch 23, mikroinjiziert mit einem replikationsdefekten Retrovirusvektor mit einem Titer von nicht weniger als $1 \times 10^8$ cfu/ml.

**25.** Herstellungsverfahren eines G0-transgenen chimären Vogels gemäß Anspruch 24, mikroinjiziert mit einem replikationsdefekten Retrovirusvektor mit einem Titer von nicht weniger als $1 \times 10^9$ cfu/ml.

**26.** Herstellungsverfahren eines G0-transgenen chimären Vogels gemäß einem der Ansprüche 20 bis 25, wobei der Retrovirusvektor ein Vektor ist, welcher vom Moloney-Mäuse-Leukämie-Virus stammt.

**27.** Herstellungsverfahren eines G0-transgenen chimären Vogels gemäß einem der Ansprüche 20 bis 26, wobei der Retrovirusvektor ein VSV-G-Pseudotyp ist.

**28.** Herstellungsverfahren eines G0-transgenen chimären Vogels gemäß einem der Ansprüche 20 bis 27, wobei der Vogel ein Huhn oder eine Wachtel ist.

**29.** Herstellungsverfahren eines G0-transgenen chimären Vogels gemäß den Ansprüchen 20 bis 28, wobei die Gensequenz, welche das Antikörpergen kodiert, durch ein Hühner-beta-Aktin-Promotor kontrolliert wird.

**30.** Herstellungsverfahren eines G0-transgen chimären Vogels gemäß einem der Ansprüche 20 bis 29, wobei das Antikörpergen ein chimäres Antikörpergen ist.

**31.** Herstellungsverfahren eines G0-transgen chimären Vogels gemäß einem der Ansprüche 20 bis 30, wobei das Antikörpergen ein scFv-Fc-Antikörpergen ist.

**32.** Herstellungsverfahren eines Antikörpers, welches die Herstellung des G0-transgenen chimären Vogels durch das Herstellungsverfahren gemäß einem der Ansprüche 20 bis 31 umfasst und das Gewinnen des Antikörpers aus dem Blut und/oder einem Ei des G0-transgenen chimären Vogels.

**33.** G0-transgener chimärer Vogel, welcher durch das Verfahren gemäß einem der Ansprüche 20 bis 31 hergestellt wird, wobei die Menge des Proteins, welches von dem Transgen stammt, nicht weniger als 20 μg/ml im Blut oder nicht weniger 5 μg/ml im Albumen ist.

**34.** Herstellungsverfahren eines transgenen Vogels, welches das Paaren des G0-transgenen chimären Vogels gemäß Anspruch 33 mit einem Paarungstyp-allogenen Vogel umfasst und anschließend das Ausschlüpfen des Eis.

**35.** Herstellungsverfahren eines transgenen Vogels gemäß Anspruch 34, wobei der Paarungstyp-allogene Vogel ein G0-transgener Vogel gemäß Anspruch 33 ist oder ein Nachkomme hiervon.

**36.** Herstellungsverfahren eines transgenen Vogels, welches weiteres Paaren des G1-transgenen Vogels, welcher durch das Verfahren gemäß Anspruch 34 erhalten wird, umfasst und das anschließende Ausschlüpfen des Eis.

**37.** Transgener Vogel oder ein Nachkomme hiervon, welcher durch das Verfahren gemäß einem der Ansprüche 34 bis 36 erhältlich ist, wobei die Menge des Antikörpers nicht weniger als 20 μg/ml im Blut oder nicht weniger als 5 μg/ml im Albumen ist.

**38.** Transgener Vogel gemäß Anspruch 37, welcher ein G1-transgener Vogel ist,
wobei der Paarungstyp-allogene Vogel der G0-transgene Vogel gemäß Anspruch 33 oder ein Nachkomme hiervon ist.

**39.** Herstellungsverfahren von einem Protein, welches das Extrahieren des Ziel-Proteins aus einer somatischen Zelle, Blut oder einem Ei des transgenen Vogels, hergestellt durch das Verfahren gemäß den Ansprüchen 34 bis 36, umfasst.

**40.** Ei, gelegt von dem transgenen Vogel gemäß den Ansprüchen 37 oder 38, welches nicht weniger als 1 mg von einem heterogenen Protein, welches von dem Transgen stammt, enthält.

**41.** Ei gemäß Anspruch 40, welches nicht weniger als 20 mg von einem heterogenen Protein, welches von dem Transgen stammt, enthält.

**42.** Ei gemäß Anspruch 41, welches nicht weniger als 100 mg von einem heterogenen Protein, welches von dem Transgen stammt, enthält.

**43.** Ei gemäß Anspruch 42, welches nicht weniger als 200 mg von einem heterogenen Protein, welches von dem Transgen stammt, enthält.

## Revendications

**1.** Oiseau chimère transgénique GO dans lequel a été introduit un gène d'anticorps exogène avec un vecteur rétroviral défectueux pour la réplication, et qui produit un anticorps dérivé du transgène dans le sang, l'albumen et/ou le jaune d'oeuf, où la teneur en ledit anticorps est d'au moins 20 μg/ml dans le sang, ou d'au moins 5 g/ml dans l'albumen.

**2.** Oiseau chimère transgénique GO selon la revendication 1, où une classe d'une région constante de l'anticorps est celle des IgG humaines.

**3.** Oiseau chimère transgénique GO selon la revendication 1, où une sous-classe d'une région constante de l'anticorps est celle des IgG1 humaines.

**4.** Oiseau chimère transgénique GO selon la revendication 1, où la région constante de l'anticorps est une IgG de caille, une IgG de poulet ou une IgG de souris.

**5.** Oiseau chimère transgénique G0 selon l'une quelconque des revendications 1 à 4, où le gène de l'anticorps est contrôlé par un promoteur constitutif.

**6.** Oiseau chimère transgénique GO selon la revendication 5, où le promoteur constitutif est le promoteur de la bêta-actine de poulet.

**7.** Oiseau chimère transgénique GO selon l'une quelconque des revendications 1 à 6, où le vecteur rétroviral est un vecteur dérivé du virus de la leucémie murine de Moloney.

8. Oiseau chimère transgénique GO selon l'une quelconque des revendications 1 à 7, où le vecteur rétroviral est un vecteur de pseudotype VSV-G.

9. Oiseau chimère transgénique GO selon l'une quelconque des revendications 1 à 8, l'oiseau étant un poulet ou une caille.

10. Oiseau chimère transgénique GO selon l'une quelconque des revendications 1 à 9, où l'anticorps est un anticorps chimère.

11. Oiseau chimère transgénique G0 selon la revendication 10, qui contient au moins 0,1 μg/ml de l'anticorps dans le jaune d'oeuf.

12. Oiseau chimère transgénique GO selon la revendication 11, qui contient au moins 1 μg/ml de l'anticorps dans le jaune d'oeuf.

13. Oiseau chimère transgénique GO selon l'une quelconque des revendications 1 à 9, où l'anticorps est un anticorps scFv-Fc.

14. Oiseau chimère transgénique GO selon la revendication 13, qui contient au moins 20 μg/ml de l'anticorps dans le sang.

15. Oiseau chimère transgénique GO selon la revendication 14, qui contient au moins 2000 μg/ml de l'anticorps dans le sang.

16. Oiseau chimère transgénique GO selon la revendication 13, qui contient au moins 5 μg/ml de l'anticorps dans l'albumen.

17. Oiseau chimère transgénique GO selon la revendication 16, qui contient au moins 500 μg/ml de l'anticorps dans l'albumen.

18. Oiseau chimère transgénique GO selon la revendication 13, qui contient au moins 5 μg/ml de l'anticorps dans le jaune d'oeuf.

19. Oiseau chimère transgénique GO selon la revendication 18, qui contient au moins 500 μg/ml de l'anticorps dans le jaune d'oeuf.

20. Procédé de production d'un oiseau chimère transgénique G0, qui comprend l'incubation d'un oeuf fertile d'oiseau, l'infection d'un embryon précoce, après un délai d'au moins 24 heures à partir du début de l'incubation, avec un vecteur rétroviral défectueux pour la réplication, puis l'éclosion de l'embryon,
où une séquence de gène codant pour un gène d'anticorps est contenue dans un transgène incorporé dans un vecteur rétroviral défectueux pour la réplication.

21. Procédé de production d'un oiseau chimère transgénique GO selon la revendication 20, qui comprend l'incubation d'un oeuf fertile d'oiseau, ayant subi une micro-injection d'un vecteur rétroviral défectueux pour la réplication dans le coeur ou un vaisseau sanguin formé dans l'embryon précoce.

22. Procédé de production d'un oiseau chimère transgénique GO selon la revendication 20, qui comprend l'incubation d'un oeuf fertile d'oiseau, ayant subi une micro-injection d'un vecteur rétroviral défectueux pour la réplication dans le coeur ou un vaisseau sanguin formé dans l'embryon précoce formé après un délai d'au moins 24 heures à partir du début de l'incubation.

23. Procédé de production d'un oiseau chimère transgénique G0 selon l'une quelconque des revendications 20 à 22, ayant subi une micro-injection d'un vecteur rétroviral défectueux pour la réplication ayant un titre d'au moins $1 \times 10^7$ ufc/ml.

24. Procédé de production d'un oiseau chimère transgénique GO selon la revendication 23, ayant subi une micro-injection d'un vecteur rétroviral défectueux pour la réplication ayant un titre d'au moins $1 \times 10^8$ ufc/ml.

25. Procédé de production d'un oiseau chimère transgénique GO selon la revendication 24, ayant subi une micro-injection d'un vecteur rétroviral défectueux pour la réplication ayant un titre d'au moins $1 \times 10^9$ ufc/ml.

26. Procédé de production d'un oiseau chimère transgénique GO selon l'une quelconque des revendications 20 à 25, où le vecteur rétroviral est un vecteur dérivé du virus de la leucémie murine de Moloney.

27. Procédé de production d'un oiseau chimère transgénique GO selon l'une quelconque des revendications 20 à 26, où le vecteur rétroviral est un vecteur de pseudotype VSV-G.

28. Procédé de production d'un oiseau chimère transgénique GO selon l'une quelconque des revendications 20 à 27, où l'oiseau est un poulet ou une caille.

29. Procédé de production d'un oiseau chimère transgénique GO selon l'une quelconque des revendications 20 à 28, où la séquence de gène codant pour un gène d'anticorps est contrôlée par le promoteur de la bêta-actine de poulet.

30. Procédé de production d'un oiseau chimère transgénique GO selon l'une quelconque des revendications 20 à 29, où le gène d'anticorps est un gène d'anticorps chimère.

31. Procédé de production d'un oiseau chimère transgénique GO selon l'une quelconque des revendications 20 à 30, où le gène d'anticorps est un gène d'anticorps scFv-Fc.

32. Procédé de production d'un anticorps, qui comprend la production de l'oiseau chimère transgénique GO par le procédé de production selon l'une quelconque des revendications 20 à 31, et la récupération de l'anticorps à partir du sang et/ou d'un oeuf dudit oiseau chimère transgénique G0.

33. Oiseau chimère transgénique GO qui est produit par le procédé selon l'une quelconque des revendications 20 à 31, où la teneur de la protéine dérivée du transgène est d'au moins 20 μg/ml dans le sang, ou d'au moins 5 μg/ml dans l'albumen.

34. Procédé de production d'un oiseau transgénique, qui comprend le croisement de l'oiseau chimère transgénique GO selon la revendication 33 avec un oiseau allogénique pour le type sexuel, puis l'éclosion de l'oeuf.

35. Procédé de production d'un oiseau transgénique selon la revendication 34, où l'oiseau allogénique pour le type sexuel est l'oiseau transgénique GO selon la revendication 33 ou un de ses descendants.

36. Procédé de production d'un oiseau transgénique, qui comprend en outre le croisement de l'oiseau transgénique G1 qui est obtenu par le procédé selon la revendication 34, puis l'éclosion de l'oeuf.

37. Oiseau transgénique ou sa descendance, qui peut être obtenu par le procédé selon les revendications 34 à 36, où la teneur de l'anticorps est d'au moins 20 μg/ml dans le sang, ou d'au moins 5 μg/ml dans l'albumen.

38. Oiseau transgénique selon la revendication 37, qui est un oiseau transgénique G1, où l'oiseau allogénique pour le type sexuel est l'oiseau transgénique GO selon la revendication 33 ou un de ses descendants.

39. Procédé de production d'une protéine, qui comprend l'extraction de la protéine en question d'une cellule somatique, du sang ou d'un oeuf de l'oiseau transgénique produit par le procédé selon les revendications 34 à 36.

40. Oeuf pondu par l'oiseau transgénique selon les revendications 37 ou 38, qui contient au moins 1 mg d'une protéine hétérogène dérivée d'un transgène.

41. Oeuf selon la revendication 40, qui contient au moins 20 mg d'une protéine hétérogène dérivée d'un transgène.

42. Oeuf selon la revendication 41, qui contient au moins 100 mg d'une protéine hétérogène dérivée d'un transgène.

43. Oeuf selon la revendication 42, qui contient au moins 200 mg d'une protéine hétérogène dérivée d'un transgène.

Fig. 1

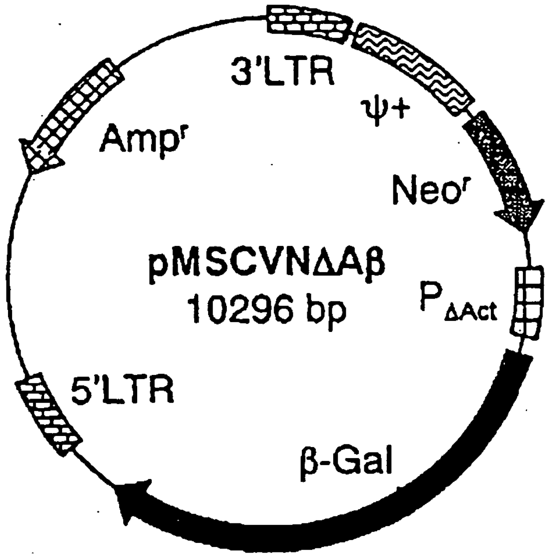
3'LTR
ψ+
Amp^r
Neo^r
pMSCVNΔAβ
10296 bp
P ΔAct
5'LTR
β-Gal

Fig. 2

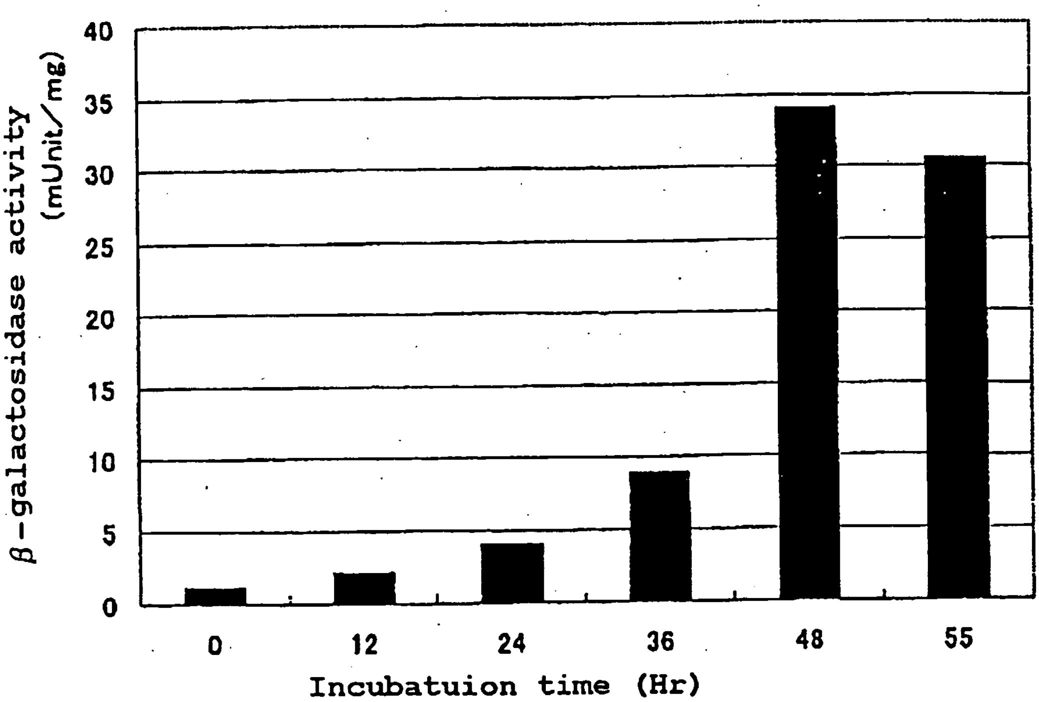
β-galactosidase activity (mUnit/mg)
40
35
30
25
20
15
10
5
0
0
12
24
36
48
55
Incubatuion time (Hr)

Fig. 3

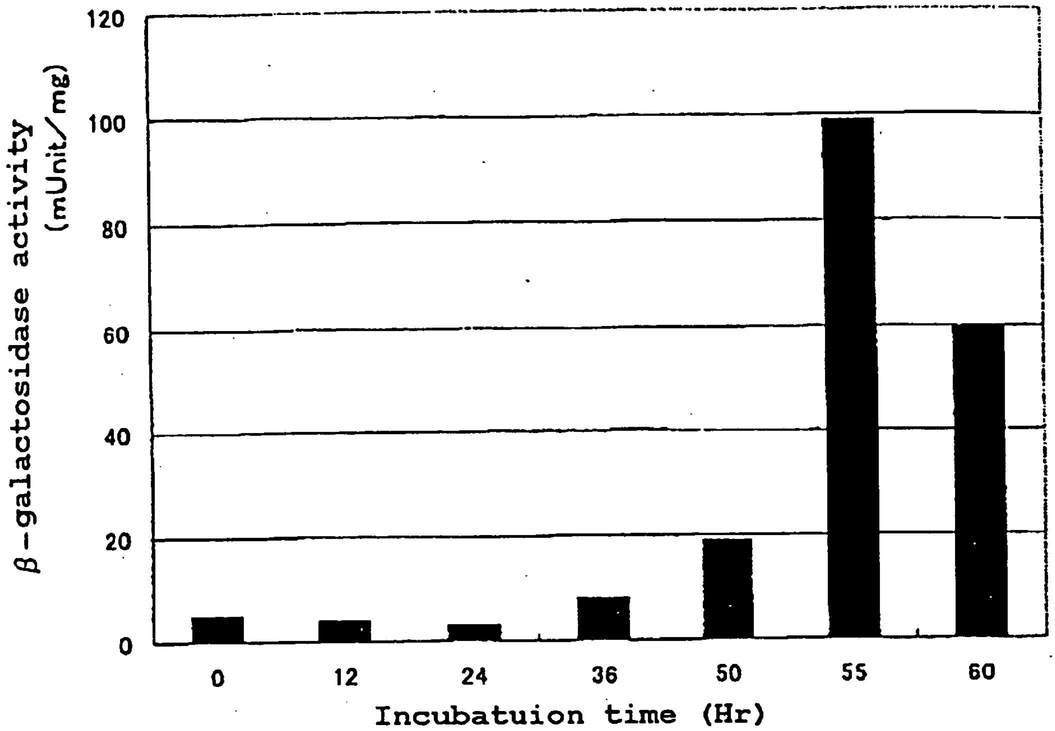
120
100
80
60
40
20
0
β-galactosidase activity (mUnit/mg)
0
12
24
36
50
55
60
Incubatuion time (Hr)

Fig. 4

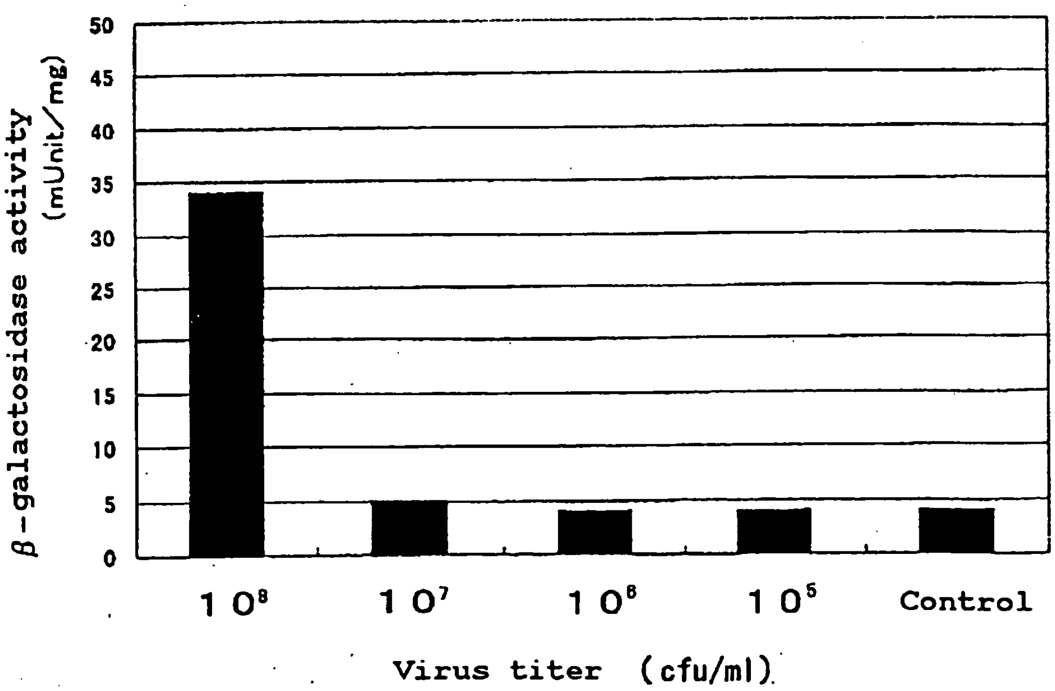
50
45
40
35
30
25
20
15
10
5
0
β-galactosidase activity (mUnit/mg)
10⁸
10⁷
10⁶
10⁵
Control
Virus titer (cfu/ml)

Fig. 5

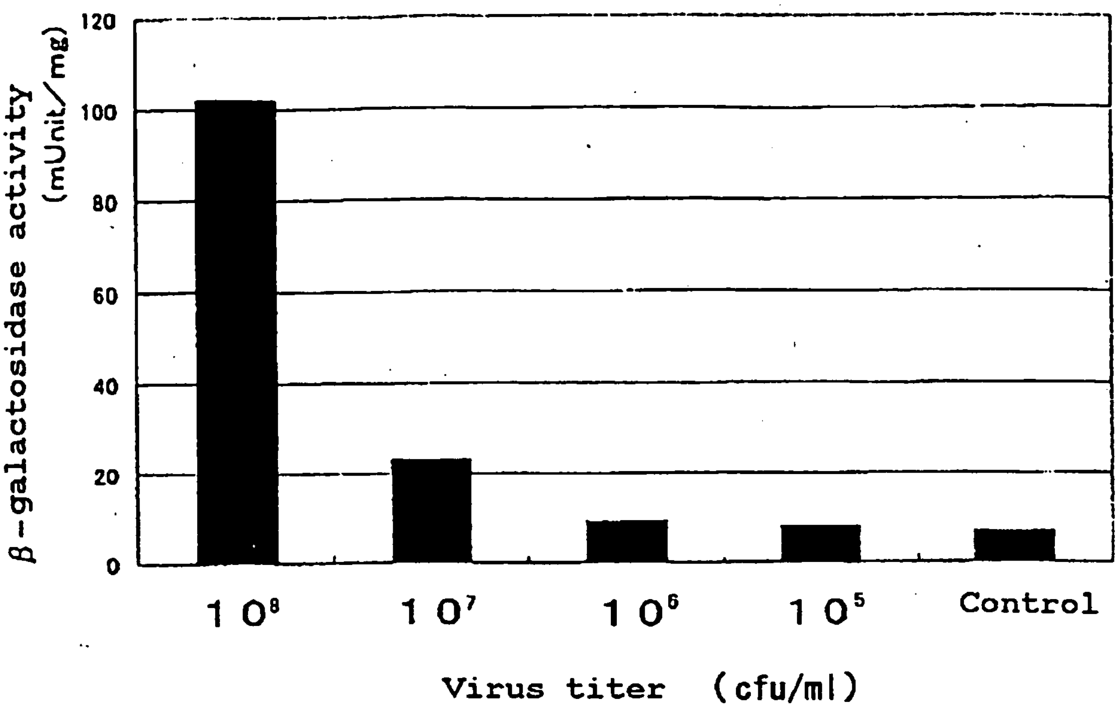
β-galactosidase activity (mUnit/mg)
120
100
80
60
40
20
0
10⁸
10⁷
10⁶
10⁵
Control
Virus titer (cfu/ml)

Fig. 6

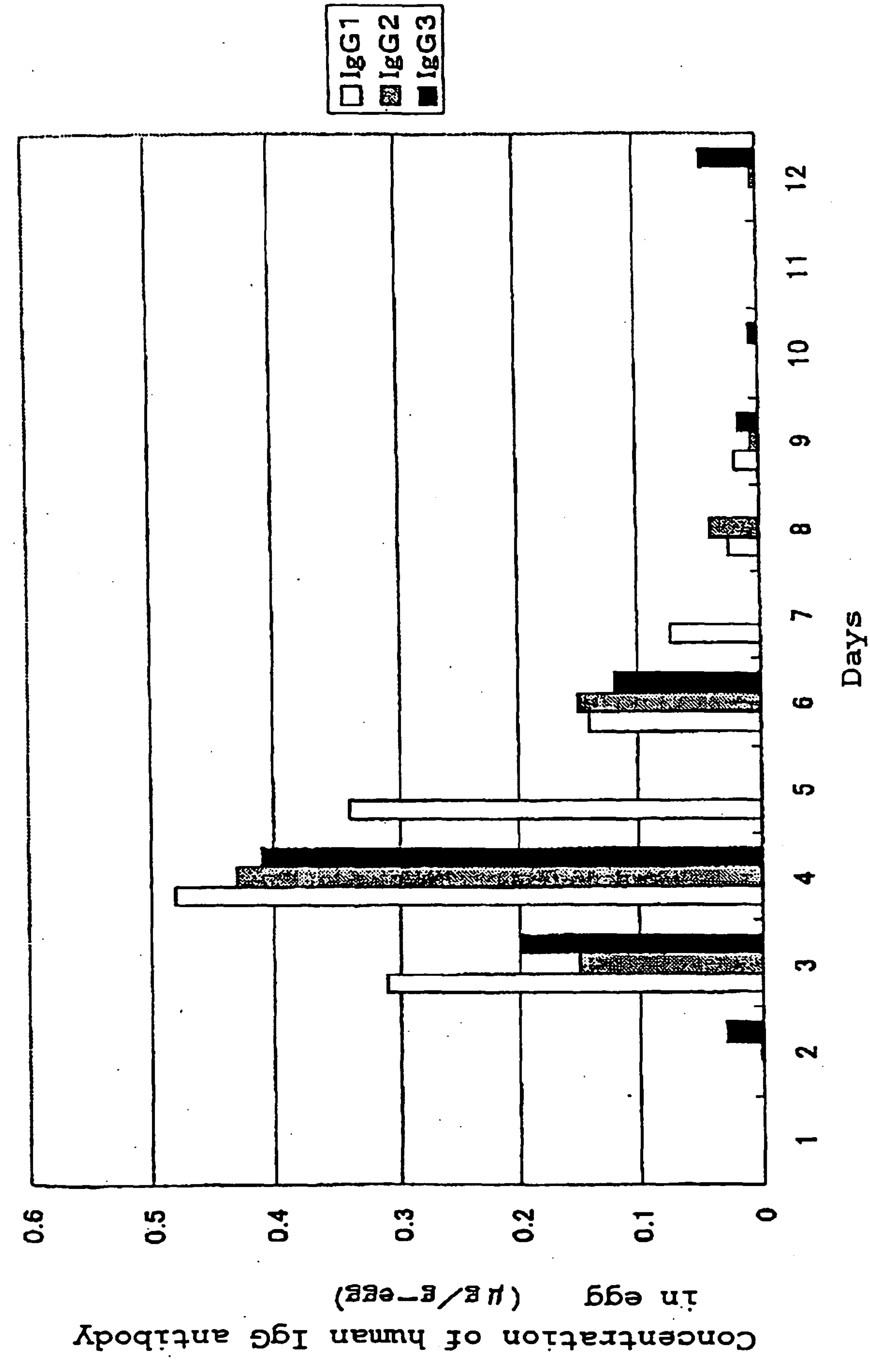
IgG1
IgG2
IgG3
0.6
0.5
0.4
0.3
0.2
0.1
0
1
2
3
4
5
6
7
8
9
10
11
12
Days
Concentration of human IgG antibody
in egg (μg/g-egg)

Fig. 7

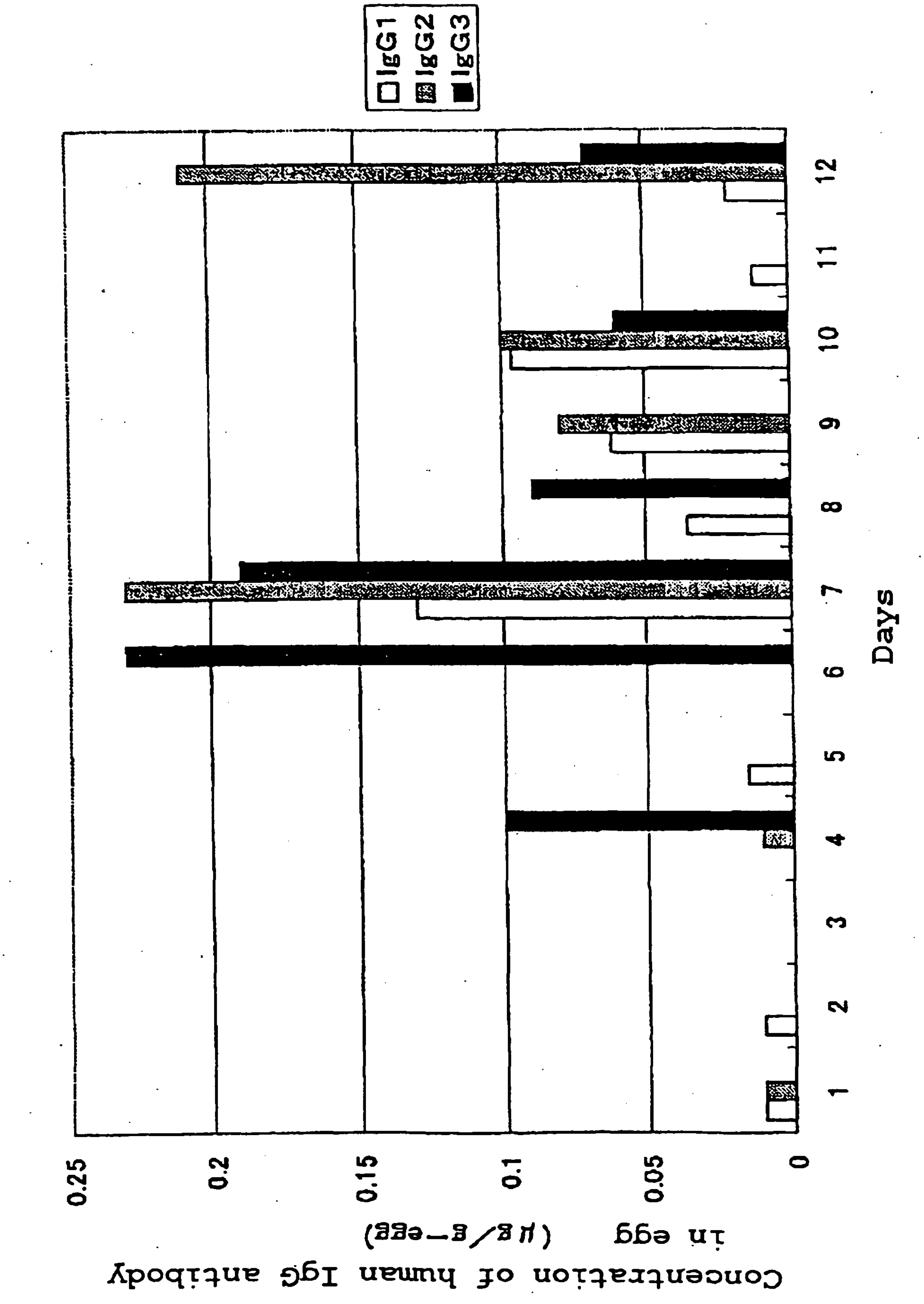
IgG1
IgG2
IgG3
Concentration of human IgG antibody in egg (μg/g-egg)
0
0.05
0.1
0.15
0.2
0.25
Days
1
2
3
4
5
6
7
8
9
10
11
12

Fig. 8

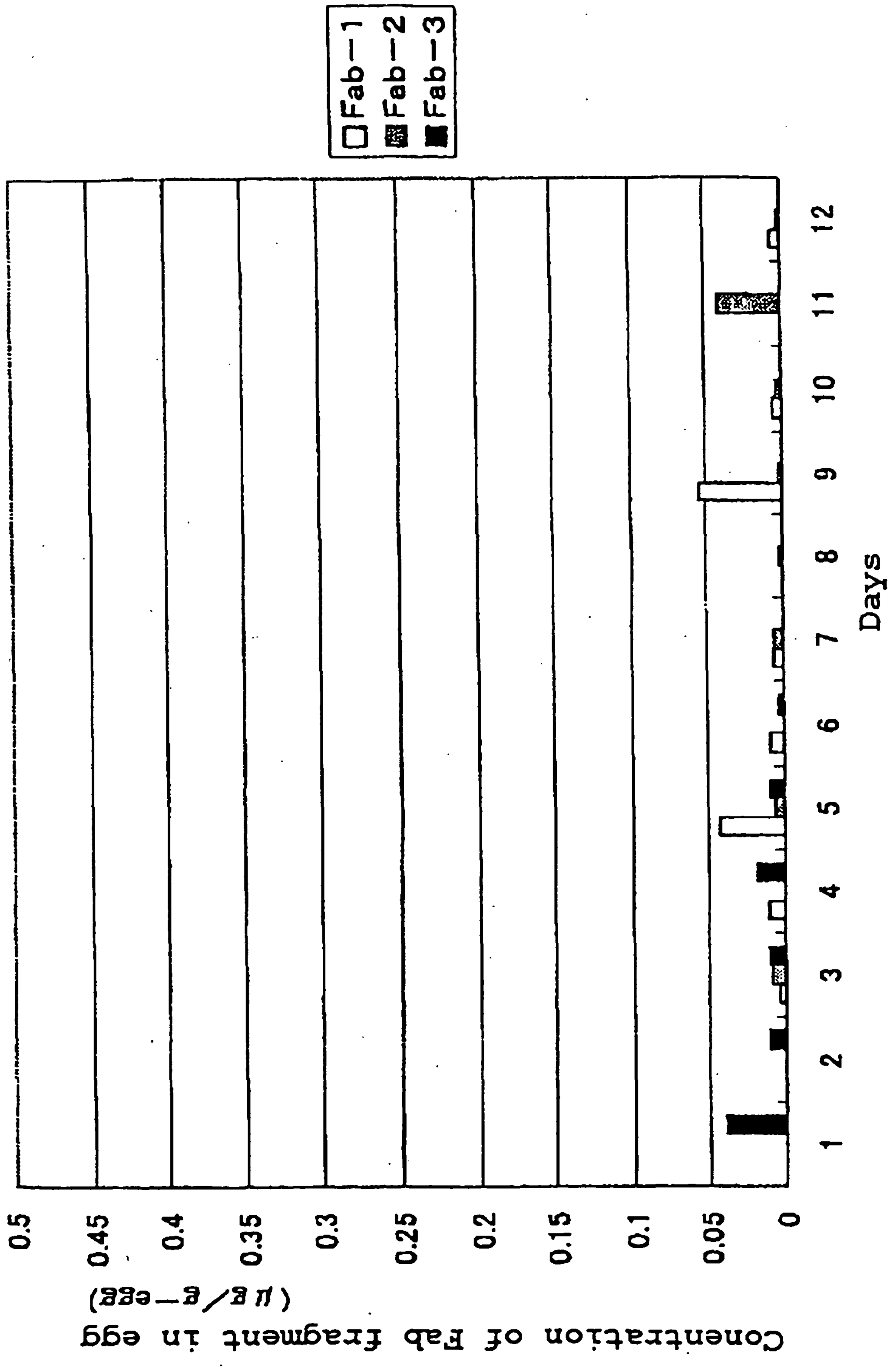
Fab—1
Fab—2
Fab—3
0.5
0.45
0.4
0.35
0.3
0.25
0.2
0.15
0.1
0.05
0
1
2
3
4
5
6
7
8
9
10
11
12
Days
Conentration of Fab fragment in egg
(μg/g-egg)

Fig. 9

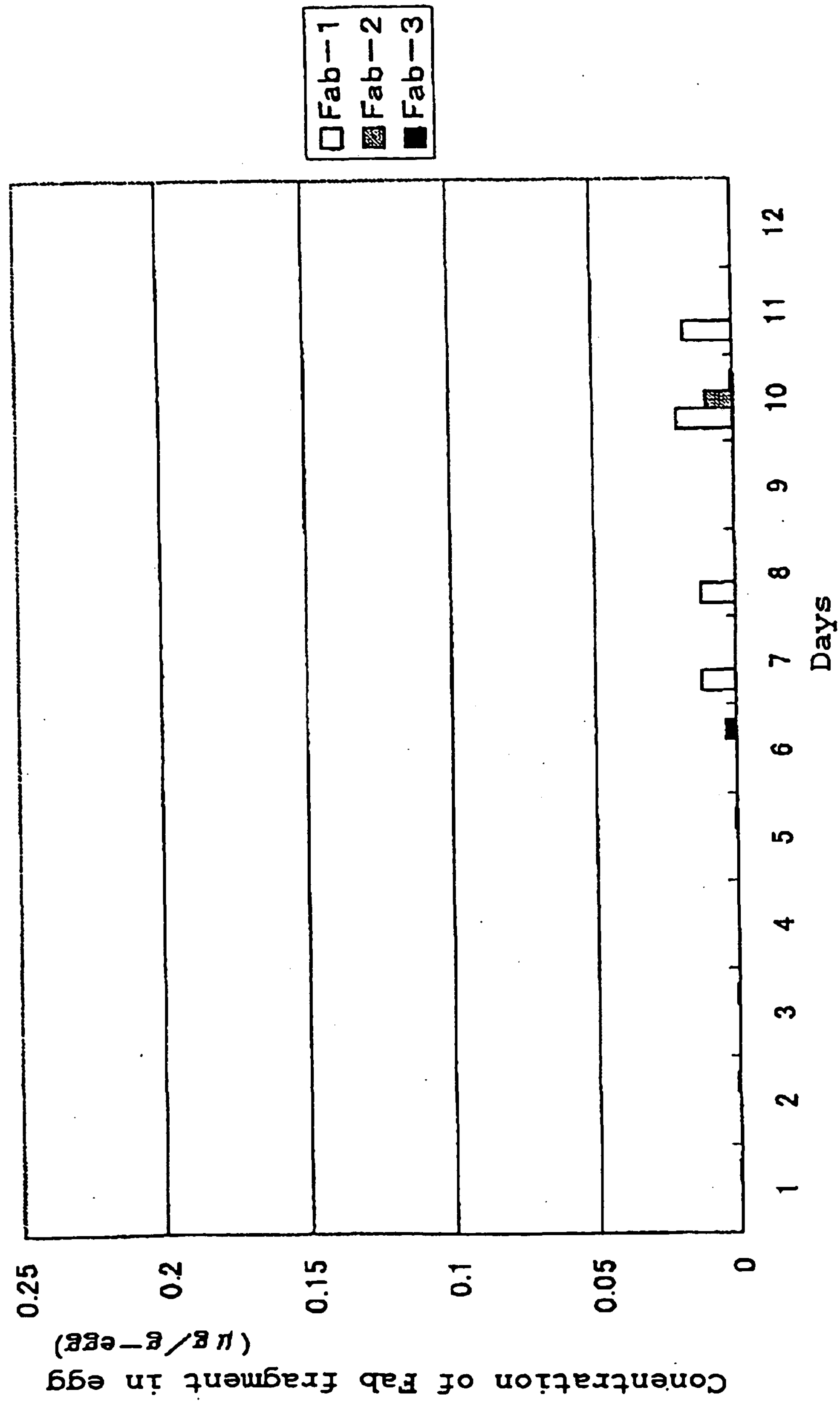
Fab-1
Fab-2
Fab-3
0.25
0.2
0.15
0.1
0.05
0
1
2
3
4
5
6
7
8
9
10
11
12
Days
Conentration of Fab fragment in egg
(μg/g-egg)

Fig. 10

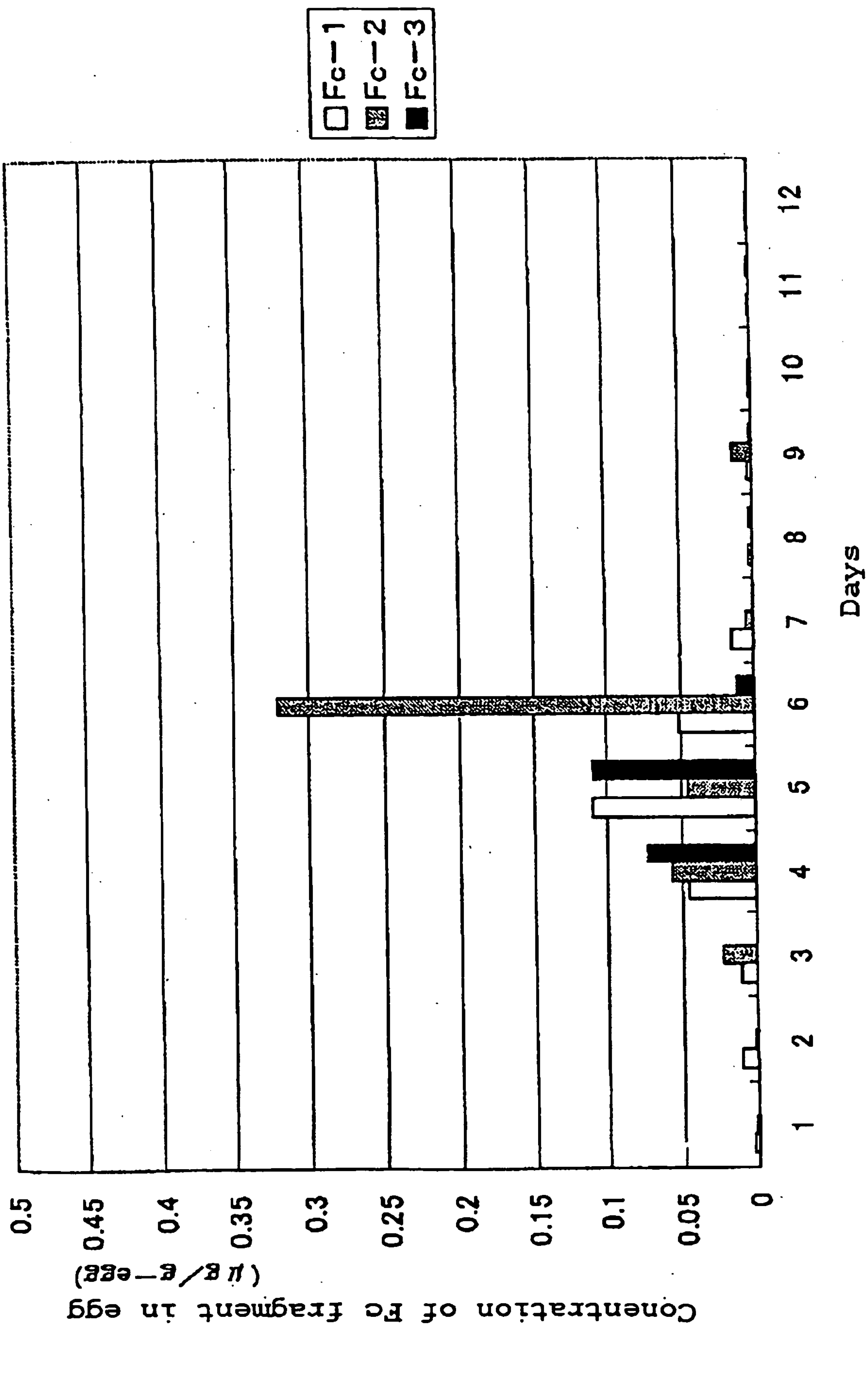
Fc—1
Fc—2
Fc—3
0.5
0.45
0.4
0.35
0.3
0.25
0.2
0.15
0.1
0.05
0
1
2
3
4
5
6
7
8
9
10
11
12
Days
Conentration of Fc fragment in egg
(μg/g—egg)

Fig. 11

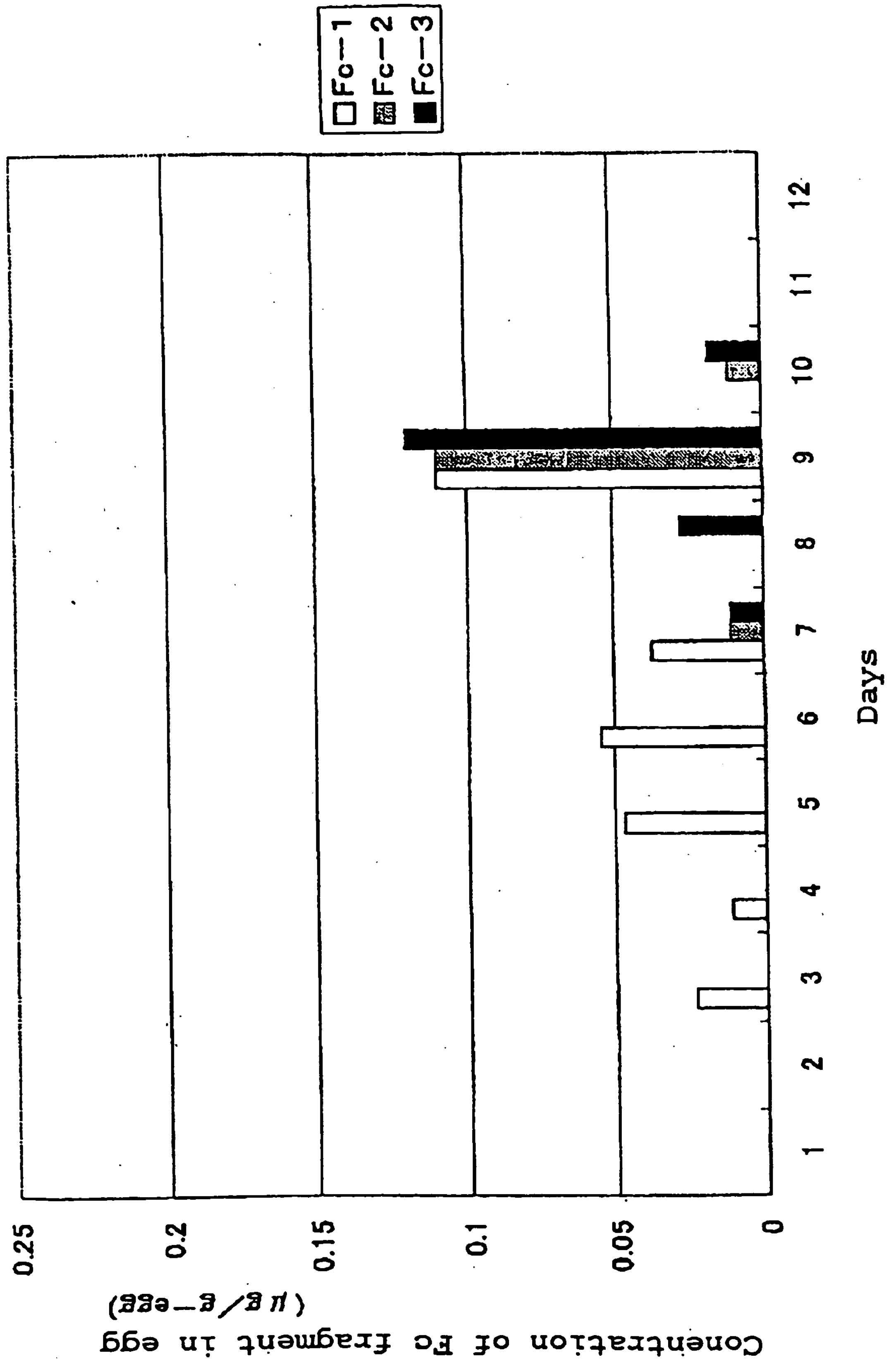
Fc—1
Fc—2
Fc—3
0.25
0.2
0.15
0.1
0.05
0
1
2
3
4
5
6
7
8
9
10
11
12
Days
Conentration of Fc fragment in egg
(μg/g—egg)

Fig. 12

(A)

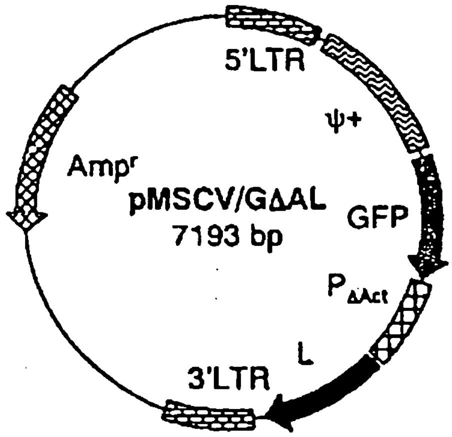
5'LTR
ψ+
Amp^r
pMSCV/GΔAL
7193 bp
GFP
P_ΔAct
L
3'LTR

(B)

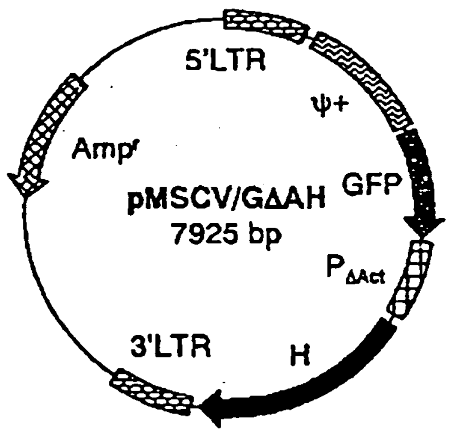
5'LTR
ψ+
Amp^r
pMSCV/GΔAH
7925 bp
GFP
P_ΔAct
3'LTR
H

(C)

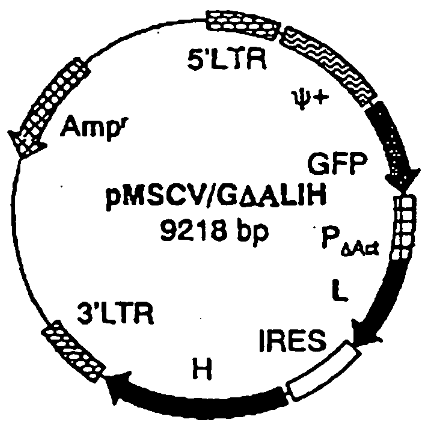
5'LTR
ψ+
Amp^r
GFP
pMSCV/GΔALIH
9218 bp
P_ΔAct
L
3'LTR
IRES
H

Fig. 13

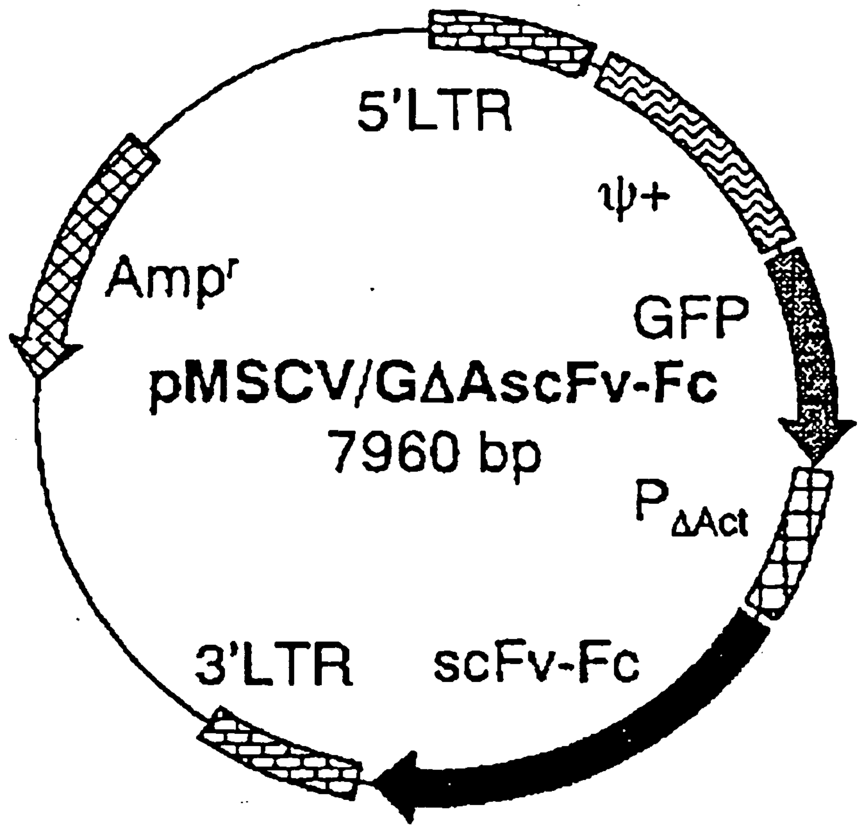

Fig. 14

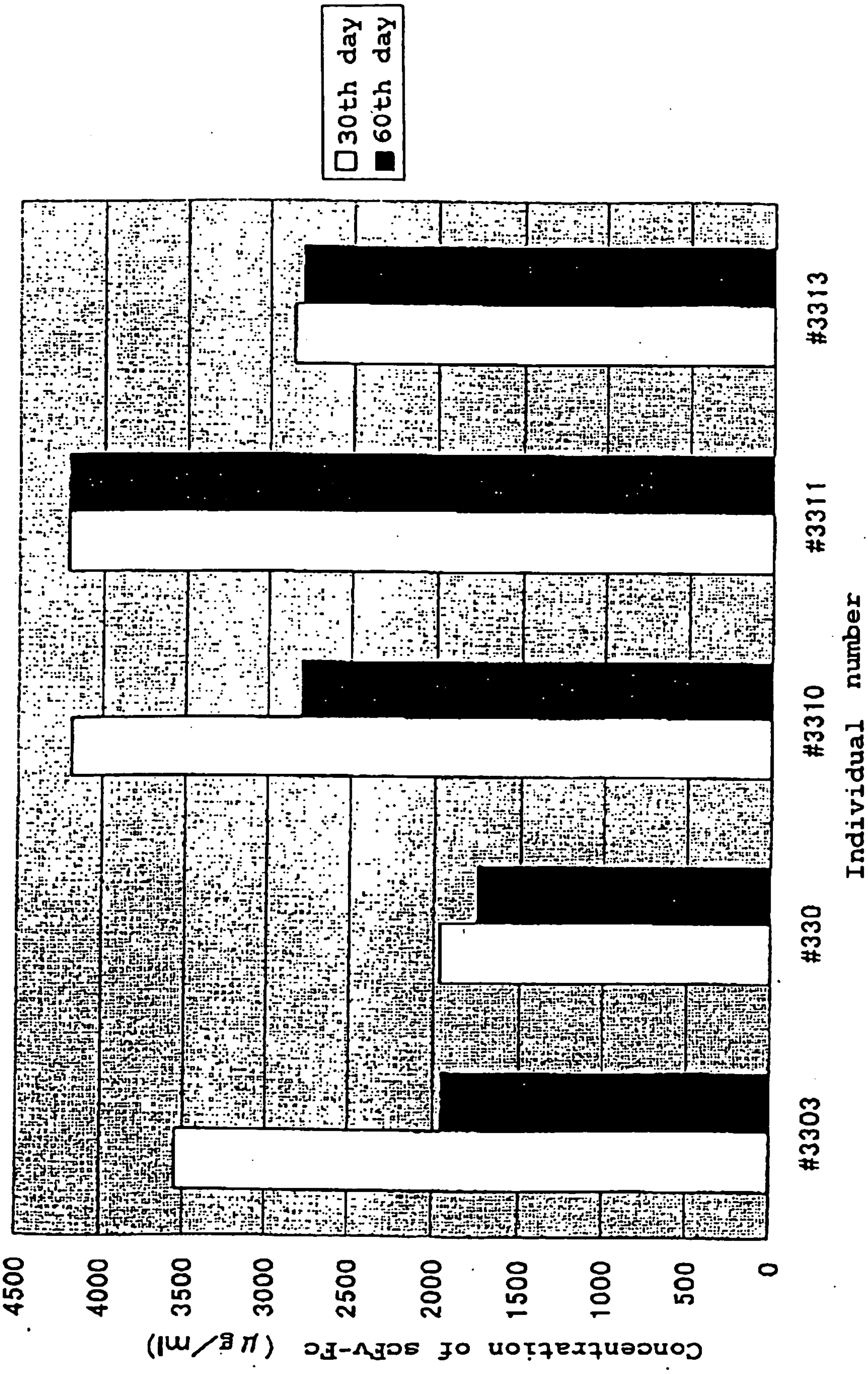
30th day
60th day
Concentration of scFv-Fc (μg/ml)
4500
4000
3500
3000
2500
2000
1500
1000
500
0
#3303
#330
#3310
#3311
#3313
Individual number

Fig. 15

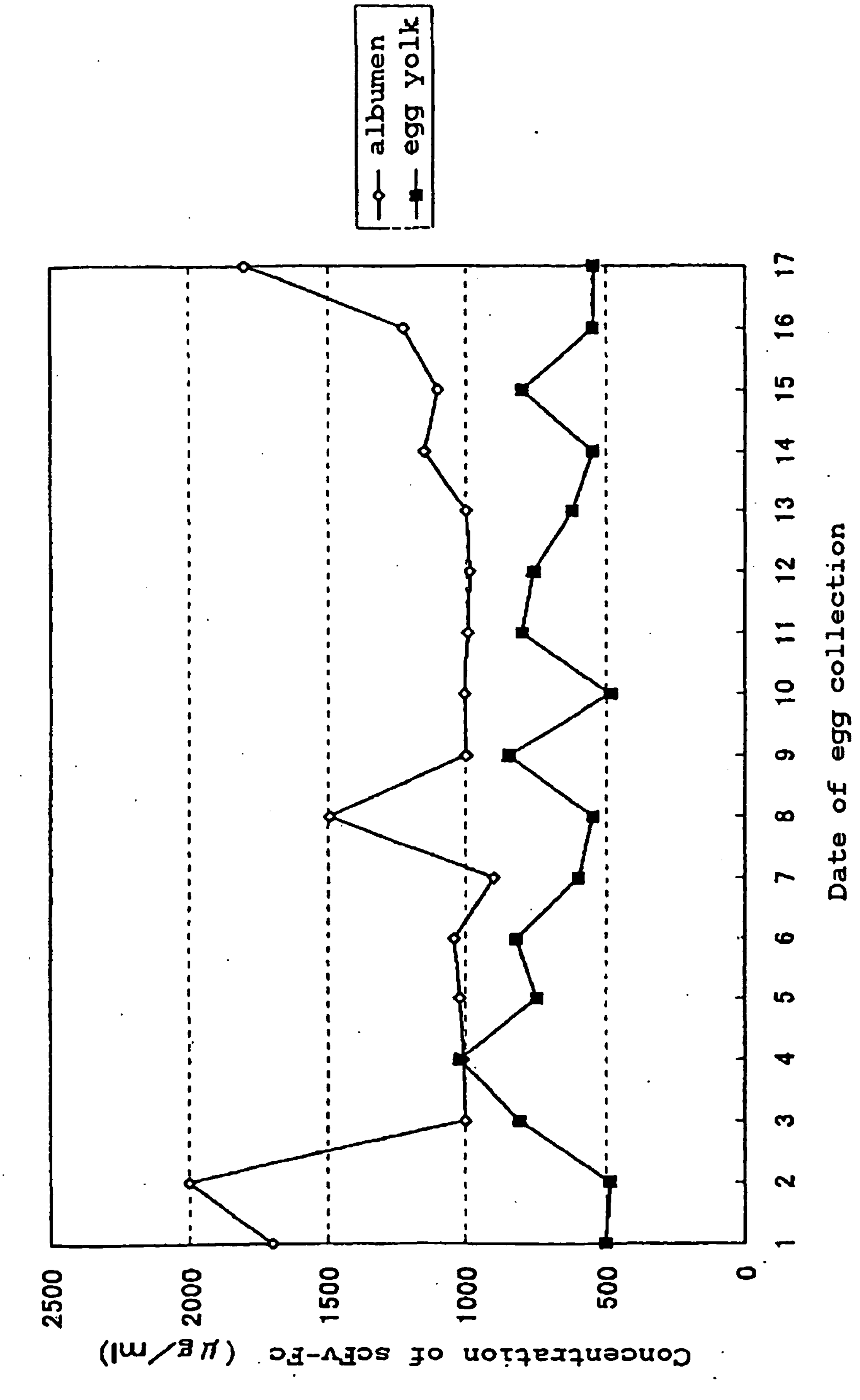
albumen
egg yolk
Concentration of scFv-Fc (μg/ml)
0
500
1000
1500
2000
2500
Date of egg collection
1
2
3
4
5
6
7
8
9
10
11
12
13
14
15
16
17

Fig. 16

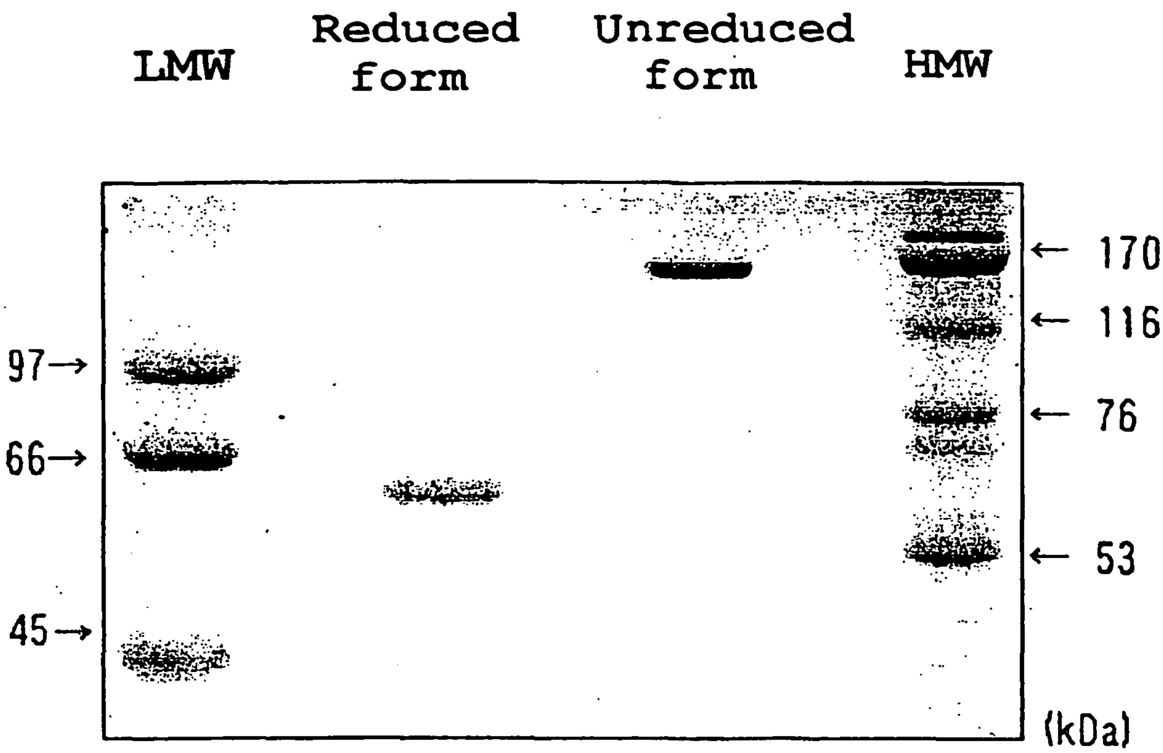

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002176880 A
- JP P2002236089 B


### Non-patent literature cited in the description


- **Powers, D.B et al.** *J Immunol Method.,* 2000, vol. 251, 123
- **Kamihira, M. et al.** *Develop. Growth Differ.,* 1998, vol. 40, 449
- **Suemori et al.** *Cell Diff. Dev.,* 1990, vol. 29, 181-185
- **Nakamura et al.** *Cytotechnology,* 2000, vol. 32, 191-198